(19) Europäisches Patentamt
European Patent Office
Office européen des brevets

(11) Publication number: **0 417 477 B1**

(12) # EUROPEAN PATENT SPECIFICATION

(45) Date of publication of patent specification: **18.10.95**

(51) Int. Cl.6: **C08F 4/22**, C08F 10/00

(21) Application number: **90115317.1**

(22) Date of filing: **09.08.90**

(54) **Process for olefin polymerization.**

(30) Priority: **10.08.89 US 393354**

(43) Date of publication of application:
**20.03.91 Bulletin 91/12**

(45) Publication of the grant of the patent:
**18.10.95 Bulletin 95/42**

(84) Designated Contracting States:
**AT BE CH DE DK ES FR GB GR IT LI LU NL SE**

(56) References cited:
**EP-A- 0 250 999**
**US-A- 3 887 494**

**CHEMICAL ABSTRACTS, vol. 112, no. 8, 19 February 1990, Columbus, Ohio, US; abstract no. 56753W, W.K.REAGEN: 'CHROMIUM(II) AND -(III) PYRROLYL ETHYLENE OLIGOMERIZATION CATALYSTS. SYNTHESIS AND CRYSTAL STRUCTURE OF SOUARE PLANAR [CR(NC4H4)4]-2, AND PENTANUCLEAR CR5(NC4H4)10(OC4H8)4'**

**JOURNAL OF THE CHEMICAL SOCIETY, CHEMICAL COMMUNICATIONS. no. 12, 15 June 1989, LETCHWORTH GB pages 674 - 675; J.R.BRIGGS: 'THE SELECTIVE TRIMERIZATION OF ETHYLENE TO HEX-1-ENE**

(73) Proprietor: **PHILLIPS PETROLEUM COMPANY**
**5th and Keeler**
**Bartlesville**
**Oklahoma 74004 (US)**

(72) Inventor: **Reagen, William Kevin**
**Rt. 1 Box 294**
**Dewey, OK 74029 (US)**

(74) Representative: **Dost, Wolfgang, Dr.rer.nat., Dipl.-Chem. et al**
**Patent- und Rechtsanwälte**
**Bardehle . Pagenberg . Dost . Altenburg . Frohwitter . Geissler & Partner**
**Postfach 86 06 20**
**D-81633 München (DE)**

EP 0 417 477 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

Rank Xerox (UK) Business Services
(3.10/3.09/3.3.3)

## Description

### BACKGROUND OF THE INVENTION

This invention relates to the polymerization of olefins, such as ethylene, in the presence of any polymerization catalyst and one or more novel chromium cocatalysts.

It is well known that olefins, such as ethylene, can be polymerized with catalyst systems employing supported, chromium catalysts or magnesium/titanium catalysts. Initially such catalysts were used primarily to form olefin homopolymers. It soon developed, however, that many applications required polymers which were more impact resistant than olefin homopolymers. Consequently, in order to produce polymer having short chain branching like the more flexible free radical polymerised olefin polymers, comonomers such as propylene, butene, hexene or the higher olefins were copolymerized with the olefin monomer to provide resins tailored to specific end uses. The copolymers, however, are more expensive to produce since inventories of different monomers must be kept and also the comonomers are generally more expensive than the usual short-chain monomers, such as ethylene or propylene. Linear olefin polymers, such as ethylene, with short chain branching can be formed from a pure ethylene feed using the old free radical high pressure process, but the conditions necessary to do this make the product too expensive to he commercially competitive.

Additional control over the polymerization process and the resultant polymer is also desired. A process to consistently reduce the density of linear olefin polymers and to more efficiently produce and incorporate comonomers into the linear olefin polymer is economically advantageous. A shift in the polymer branch distribution, wherein the branch length is decreased and the amount of branching is increased, is also economically desirable.

### SUMMARY OF THE INVENTION

Accordingly it is an object of this invention to provide a low cost route to linear olefin polymers having toughness imparted by short chain branching.

It is a further object of this invention to provide a process by which olefin polymers having the properties associated with copolymers can be obtained from a pure, single olefin feed.

It is yet a further object of this invention to provide an improved polymerization process.

It is a further object of this invention to provide a novel polymerization process to control polymer density.

It is yet a further object of this invention to provide a novel polymerization process to improve comonomer production and incorporation into olefin polymers.

It is a further object of this invention to provide a novel polymerization process to shift olefin distribution.

It is a further object of this invention to provide a novel polymerization process to control polymer short chain branching.

In accordance with this invention, an essentially pure, single olefin feed is contacted under polymerization conditions with a polymerization catalyst and an olefin trimerization cocatalyst comprising the reaction product of a chromium salt, A metal amide, and an ether. Additionally, hydrogen car be introduced into the polymerization reactor in an amount sufficient to accelerate the trimerization and/or polymerization processes.

### BRIEF DESCRIPTION OF THE DRAWINGS

FIGURE I is a computer generated, simplified structural formula of a molecule of Product I, $Cr_5(NC_4H_4)_{10}(OC_4H_8)_4$, as determined by single crystal x-ray crystallography, and

FIGURE II is a further simplified structural representation of the same molecule shown in FIGURE I.

FIGURE III is a computer generated simplified structural formula of a molecule of Product III, $Cr(NC_4H_4)_4$, as determined by single crystal x-ray crystallography.

FIGURE IV is a further simplified structural representation of the same molecule shown in Figure III, however, the entire crystal lattice, with the formula $[Cr(NC_4H_4)_4][Na]_2 \cdot 2(OC_4H_8)$ is shown.

FIGURE V is a computer generated, simplified structural formula of a molecule of Product IV, $Cr(NC_4H_4)_5(OC_4H_8)$ as determined by single crystal x-ray crystallography, and

FIGURE VI is a further simplified structural representation of the same molecule shown in FIGURE V, however the entire crystal lattice, with the formula $[Cr(NC_4H_4)_5(OC_4H_8)][Na]_2 \cdot 4(OC_4H_8)$, is shown.

## DETAILED DESCRIPTION OF THE INVENTION

### Polymerization Catalyst

The inventive chromium compounds can be used either supported or unsupported as a cocatalyst with any other olefin polymerization catalyst. Generally, polymerization catalysts are considered either chromium catalysts (also known as "Phillips Catalysts") or titanium and/or vanadium-containing catalysts.

Any chromium catalyst known in the art can be used. Exemplary chromium catalysts are disclosed in U.S. Patents 3,887,494; 3,900,457; 4,053,436; 4,151,122; 4,294,724; 4,392,990; and 4,405,501.

Any titanium and/or vanadium-containing catalyst known in the art can also be used. Exemplary magnesium/titanium catalysts are disclosed in U.S. Patents 4,394,291; 4,326,988; and 4,347,158.

A preferred polymerization catalyst is a supported chromium catalyst.

The amount of inventive chromium compound used as a cocatalyst can be any amount sufficient to generate a comonomer that can be incorporated into the polymer product.

### The Chromium Compounds

The inventive chromium compounds, which can be used preferably for olefin trimerization and, optionally, olefin polymerization, can be produced by forming a reaction mixture comprising a chromium salt, a metal amide, and an ether. As used in this disclosure, the inventive chromium compounds are referred to by a variety of names, such as inventive or novel chromium compound(s), chromium complex(es), chromium pyrrole complex(es) and/or chromium pyrrolide.

The chromium salt can be one or more organic or inorganic chromium salts, wherein the chromium oxidation state is from 0 to 6. As used in this disclosure, chromium metal is included in this definition of a chromium salt. Generally, the chromium salt will have a formula of $CrX_n$. wherein X can be the same or different and can be any organic or inorganic radical, and n is an integer from 1 to 6. Exemplary organic radicals can have from 1 to 20 carbon atoms, and are selected from the group consisting of alkoxy, ester, ketone, and/or amido radicals. The organic radicals can be straight-chained or branched, cyclic or acyclic, aromatic or aliphatic, and can be made of mixed aliphatic, aromatic, and/or cycloaliphatic groups. Exemplary inorganic radicals include, but are not limited to halides, sulfates, and/or oxides.

Preferably, the chromium salt is a halide, such as, for example chromous bromide, chromic bromide, chromous iodide, chromic iodide, chromous flouride, chromic flouride, chromous chloride, chromic chloride, and mixtures thereof. Most preferably, the chromium salt is a chloride, such as, for example chromous chloride and/or chromic chloride, due to simple separation of the reaction by-products such as, for example, sodium chloride, as well as relatively low cost.

The metal amide can be any metal amide that will react a chromium salt to form a chromium-amido complex. Broadly, the metal amide can be any heteroleptic or homoleptic metal complex or salt, wherein the amide radical can be any nitrogen-containing organic radical. Generally, the metal amide will have from 1 to 20 carbon atoms. Exemplary metal amides include, but are not limited to, primary and/or secondary amines, any alkali metal (Group IA of the Periodic Table) amide and/or any alkaline earth metal (Group IIA of the Periodic Table) amide. The hydrocarbyl portion of the salt of the metal amide is selected from the group consisting of straight chain or branched, cyclic or acyclic, aromatic or aliphatic, and mixtures of two or more thereof. Preferably, the metal amid is selected from a Group IA or Group IIA metal amide, due to ease of reaction with chromium halides. Exemplary preferred metal amides include, but are not limited to, lithium dimethylamide, lithium diethylamide, lithium diisopropylamide, lithium dicyclohexylamide, sodium bis(trimethylsilyl)amide, sodium indolide, sodium pyrrolide, and mixtures of two or more thereof. Most preferably, the metal amide is selected from the group consisting of sodium pyrrolide, lithium pyrrolide, and/or the salts of substituted pyrrolides, because of high reactivity and activity with the other reactants. Examples of salts of substituted pyrrolides include, but are not limited to sodium 2,5-dimethyl pyrrolide and/or 3,4-dimethyl pyrrolide.

The ether in the reaction mixture can be one or more ether compounds to affect a reaction between the chromium salt and the metal amide. While not wishing to he bound by theory, it is believed that the ether can be a reaction solvent, as well as a possible reactant. The ether can be any aliphatic and/or aromatic compound containing an R-O-R functionality, wherein the R groups can be the same or different, but is not hydrogen. Preferred ethers are aliphatic ethers, for safety reasons in that aromatic ethers are human toxins. Furthermore, the preferred ethers are those which facilitate a reaction between a chromium halide and a Group IA or Group IIA metal pyrrolide, and also can be easily removed from the reaction mixture. Exemplary compounds include, but are not limited to, tetrahydrofuran, dioxane, diethylether, dimethoxyethane, diglyme, triglyme, and mixtures of two or more thereof. Most preferably, the ether is selected from the group consisting of tetrahydrofuran, derivatives of tetrahydrofuran, dimethoxyethane, derivatives of dimethoxyethane, and mixtures thereof, for the reasons given above, as well as that the preferred salt of an amino is soluble in these ethers.

The amount of each reactant used to prepare one or more of the novel chromium compounds can vary, based on the desired chromium compound product. Any amount of each reactant can be used to produce the novel chromium compounds, depending on the desired product. Different reaction stoichiometrics can produce different chromium compounds. For example, the reaction of one mole of chromium (II) with two moles of sodium pyrrollide can produce different products than reacting one mole of chromium (II) with an excess of sodium pyrollide. Furthermore, as stated earlier, selection of different, although similar reactants, can produce different products. For example, using either tetrahydrofuran or dimethoxyethane can result in a different reaction product.

The three reactants can be combined in any manner under conditions suitable to form a solution comprising one or more of the inventive chromium compounds. The reaction preferably occurs in the absence of oxygen and therefore under an inert gas, such as, for example, nitrogen and/or argon. The reaction pressure can be any pressure sufficient to maintain the reactants in a liquid state. Generally, pressures within the range of from atmospheric pressure to three atmospheres are acceptable. For ease of operation atmospheric pressure is generally employed.

The reaction temperature can be any temperature which maintains the ether in a liquid form. In order to effectuate a more efficient reaction, temperatures near the boiling point of the ether are preferred. Most preferably, the reaction temperature is at the boiling point of the ether and the reaction mixture is refluxed for a period of time.

The reaction time can be any amount of time necessary for the reaction to occur. Depending on the reactants, as well as the reaction temperature and pressure, reaction time can vary from 1 minute to 1 week. Usually, reaction time ranges from 3 hours to 5 days. Under optimum conditions, the reaction time can be within the range of from 3 to 48 hours.

After the reaction is complete, a solid reaction product can be recovered by any method known in the art. Preferably, though not required, upon completion of the reaction, the reaction mixture first is filtered to remove any reaction by-products such as, for example, salts, like sodium chloride, prior to any other treatment. Although removal of any reaction by-products is not necessary, such removal preferably is done in order to expedite later purification of the chromium product. After filtering, one exemplary method to recover a solid reaction product is to remove the excess ether from the reaction mixture. The excess ether can be removed according to any method known in the art. Exemplary ether removal methods include, but are not limited to, slow evaporation, under vacuum and/or a nitrogen purge.

Other ether removal procedures can he used either alone or in combination. For example, the reaction mixture can be filtered and then vacuum dried. Preferably, the reaction mixture is heated slowly and maintained at temperature within the range of 10° to 300°C, preferably 25° to 200°C, under a vacuum, for safety, to remove the excess ether. The resultant solid reaction product is one or more of the inventive chromium compounds.

Alternatively, the reaction mixture can be filtered to remove any solid reaction by-product and the filtered reaction mixture can be contacted with a non-polar organic solvent. Addition of a non-polar organic solvent causes one or more of the inventive chromium compounds to form a solid precipitate. Exemplary non-polar organic solvents include, hut are not limited to, pentane, hexane, cyclohexane, heptane, and mixtures thereof. Most preferably pentane is added to the filtered reaction mixture because of the availability and ease of use.

The precipitated inventive chromium compounds can be recovered by any method known in the art. The simplest procedure to recover the inventive precipitated chromium compounds is by filtration.

The reaction mixture and the resultant solid reaction products, as stated earlier, are kept in an oxygen-free atmosphere at all times. Preferably, due to availability and ease of use, an inert atmosphere such as, for example, nitrogen, is the ambient.

Numerous chromium compounds can be prepared in accordance to the invention, by varying the reactants and/or the quantity of each reactant employed. The recovered, novel chromium compound or compounds can be used for olefin trimerization and/or polymerization without further purification.

Optionally, the chromium compound can be purified in accordance with any method known in the art. For example, one of the simplest purification procedures is to wash the recovered solid with a non-polar organic solvent such as, for example, toluene. Preferably, a non-polar aliphatic organic solvent is used for best results. Exemplary wash solvents include, but are not limited to, pentane, hexane, cyclohexane, heptane, and mixtures thereof. Most preferably, pentane is the wash solvent.

The inventive chromium compounds can be used as supported and/or unsupported catalyst for olefin trimerization and/or polymerization. A supported chromium catalyst can be prepared according to any method known in the art. Any support useful to support chromium catalyst can be used. Exemplary catalyst supports include, but are not limited to, inorganic oxides, either alone or in combination, phosphated inorganic oxides, and mixtures thereof. Particularly preferred are supports selected from the group consisting of silica, silica-alumina, alumina, fluorided alumina, silated alumina, thoria, aluminophosphate, aluminum phosphate, phosphated silica, phosphated alumina, silica-titania, coprecipitated silica/titania, and mixtures, thereof, fluorided/silated alumina, being presently preferred, as well as any one or more of these supports which can contain chromium. The presently most preferred catalyst support is because of the greatest trimerization activity, is aluminophosphate, as disclosed in U.S. Patent 4,364,855 (1982). The supported chromium catalyst system can be prepared according to any method known in the art. For example, the reaction mixture, which preferably has been filtered to remove any particulate reaction by-products and contains one or more of the novel chromium pyrrolide compounds, is combined and thoroughly contacted with a catalyst support. Excess ether does not have to be removed prior to contacting the catalyst support. However, a solid chromium pyrrolide compound can be re-dissolved in an ether, if desired. The chromium pyrrolide/ether solution is usually a blue or blue/green color. The catalyst support usually is insoluble in the ether/chromium pyrrolide complex solution. Any excess of the chromium pyrrolide in relation to the catalyst support is sufficient. However, usually, at least 5 grams of chromium pyrrolide compound per gram of catalyst support is sufficient. Preferably 0.001 to 1 grams of chromium pyrrolide compound per gram of support, and most preferably 0.01 to 0.5 grams of chromium pyrrolide compound per gram of support is used for best support loading and most efficient use of the reagents. This mixture can be contacted and mixed at any time, temperature, and pressure to thoroughly contact the chromium pyrrolide compound and support. For ease of use, ambient temperatures and pressures are preferred. Mixing times can be up to 24 hours, preferably 5 seconds to 10 hours, and most preferably 5 seconds to 8 hours. Longer times usually provide no additional benefit and shorter times can be insufficient for thorough contacting.

After the support is added and thoroughly combined with the chromium pyrrolide it is collected by filtration, vacuum dried, then a solution of one or more Lewis acids, preferably in a hydrocarbon solvent, is added to the support/chromium pyrrolide mixture. As used in this disclosure, a Lewis acid is defined as any compound that is an electron acceptor. Preferred Lewis acids include, but are not limited to, alkylaluminum compounds, derivatives of alkylaluminum compounds, halogenated alkylaluminum compounds, and mixtures thereof. Exemplary compounds include, but are not limited to, triethylaluminum, diethylaluminum, ethylaluminum sesquichloride, and mixtures thereof. The most preferred alkylaluminum compound is triethylaluminum, for best results in catalyst activity.

The hydrocarbon solvent can be any hydrocarbon that will dissolve the Lewis acid. Preferred hydrocarbons include, but are not limited to, aromatic compounds having about 6 to 50 carbon atoms per molecule. Most preferably, the hydrocarbon solvent is toluene, for ease of removal and minimal interference with the resultant catalyst.

Any amount of Lewis acid is sufficient to activate and/or react with the chromium pyrrolide catalyst. Usually 200 moles of Lewis acid per gram of chromium can be used. Preferably, 1 to 100 grams of Lewis acid per gram of chromium pyrrolide, and most preferably 5 to 30 grams of Lewis acid per gram of chromium pyrrolide are used, for best catalyst activity. However, the amount of Lewis acid employed can vary with the catalyst support used. For example, if the support is silica and/or alumina, too much Lewis acid can decrease catalyst activity. However, a similar amount of Lewis acid used with an aluminophosphate support does not always significantly decrease catalyst activity.

As disclosed earlier, the mixture of chromium pyrrolide, catalyst support, and Lewis acid are mixed and/or contacted under a dry, inert atmosphere at all times. Any pressure can be used during the contacting; for ease of use, atmospheric pressure is preferred. Any temperature can be used during the contacting; for ease of use, room temperature, or ambient temperature, is preferred. Some care should be taken during the mixing, so as not to destroy the physical integrity of the chromium pyrrolide, catalyst support, and resultant supported catalyst. The three-component mixture can he contacted for any amount of time sufficient to prepare and activate a chromium catalyst. Usually times in the range of about one minute to about one week are sufficient. Preferably, times in the range of 30 minutes to 24 hours are used, and most preferably times in the range of one hour to 12 hours are used. Too short of mixing times can result in incomplete contacting and too long of mixing times will not provide any additional catalytic benefit.

An alternative, and presently preferred, method to produce a supported catalyst is to combine one or more solid, inventive chromium pyrrolide compounds with a hydrocarbon solvent, as disclosed earlier, such as, for example, toluene, and a Lewis acid, as disclosed earlier, such as, for example, triethylaluminum. This

mixture can be stirred for any time sufficient to dissolve the chromium pyrrolide compound, at any pressure or temperature. Usually, times of one minute to one week, preferably one hour to 24 hours, and most preferably within the range of three hours to 12 hours are used. For ease of operation, ambient temperatures and pressures are used. Usually, a brown solution will result.

After the solution is sufficiently mixed, a support is added to the solution and stirred to thoroughly contact the solution and support. The quantity of support is any amount sufficient to support the chromium pyrrolide compound. Generally, the amount of support necessary is the same as that disclosed in the previous exemplary process. Any suitable pressure and temperature can be used, although ambient temperature and pressure are preferred for ease of use. Usually, the mixing and/or contacting time is within the range of 30 minutes to one week, preferably from 3 hours to 48 hours. Most preferably, the mixing and/or contacting time is within the range of 5 hours to 24 hours, to maximize efficiency and result in a thoroughly contacted support.

The solution then can be filtered to recover a solid catalytic product. The catalytic product, as with the reactants and reactions, is preferably kept under an inert atmosphere to maintain chemical stability.

If the chromium compound is recovered and is to be used as an unsupported trimerization and/or polymerization catalyst, olefins can be trimerized or polymerized in a presence of one or more of the inventive homogeneous chromium compounds, an saturated hydrocarbon, and Lewis acid. Optionally, hydrogen can be added to the reactor to accelerate the reaction.

## Reactants

Reactants applicable for use in polymerization with the catalyst and processes of this invention are olefinic compounds which can polymerize, i.e., react with the same or with other olefinic compounds. Catalyst of the invention can be used to polymerize at least one mono-1-olefin having from 2 to 30 carbon atoms, preferably from 2 to 8 carbon atoms. Exemplary compounds include, but are not limited to, ethylene, propylene, 1-butene, 1-pentene, 1-hexene, 1-octene, and mixtures thereof. A preferred reactant is ethylene, which is reacted according to the present invention to a polymer having a density in the range of 0.9 to 0.93 g/cc.

Reactants applicable for use in the trimerization process, i.e., the combination of any three olefins, of this invention are olefinic compounds which can a) self-react, i.e., trimerize, to give useful products such as, for example, the self reaction of ethylene can give one hexene; and/or b) olefinic compounds which can react with other olefinic compounds, i.e., co-trimerize, to give useful products such as, for example, co-trimerization of ethylene plus hexene can give one decene and/or 1-tetradecene, co-trimerization of ethylene and 1-butane gives one octene, or 1-decene end ethylene con give 1-tetradecene and/or 1-docosene. As used herein, the term "trimerization" is intended to include "co-trimerization" as defined above.

Suitable trimerizable olefinic compounds are those compounds having from 2 to 30 carbon atoms per molecule and having at least one olefinic double bond. Exemplary compounds include, but are not limited to acyclic and cyclic olefins such as, for example, ethylene, propylene, 1-butene, 2-butene, isobutylene, 1-pentene, 2-pentene, 1-hexene, 2-hexene, 3-hexene, 1-heptene, 2-heptene, 3-heptene, the four normal octenes, the four normal nonenes, and mixtures of any two or more thereof. If branched and/or cyclic olefins are used as reactants, while not wishing to be bound by theory, it is believed that steric hindrance could hinder tie trimerization process. Therefore, the branched and/or cyclic portion(s) of the olefin should be distant from the carbon-carbon double bond.

## Reaction Conditions

The reaction products, i.e., trimers and/or polymers, can be prepared from the catalyst systems of this invention by solution reactions, slurry reactions, and/or gas phased reaction techniques using conventional equipment and contacting processes. Contacting of the monomer or monomers with the catalyst system can be effected by any manner known in the art of solid catalyst. One convenient method is to suspend the catalyst system in an organic medium and to agitate the mixture to maintain the catalyst system in suspension throughout the trimerization and/or polymerization process. Other known contacting methods such as fluidized bed, gravitating bed, and fixed bed can also be employed. Optionally, hydrogen can be added to the reactor to accelerate the reaction.

The catalyst systems of this invention are particularly suitable for use in slurry trimerization and/or polymerizations. The slurry process is generally carried out in an inert diluent (medium), such as a paraffin, cycloparaffin, or aromatic hydrocarbon. One exemplary reactor diluent is isobutane. When the reactant is

predominately ethylene, a temperature in the range of 60° to 110°C, preferably 66 to 110°C, is employed.

## Products

The olefinic and/or polymeric products of this invention have established utility in a wide variety of application such as, for example, as monomers for use in the preparation of homopolymers, copolymers, and/or terpolymers. The polymeric products of this invention have established utility in a wide variety of application such as for example, polyethylene.

The further understanding of the present invention and its advantages will be provided by reference to the following examples.

## Examples

### Preparation of Chromium-Containing Compounds

Manipulations of all reactants were carried out either in a drybox employing nitrogen, or in airless glassware employing vacuum or nitrogen. Tetrahydrofuran (THF), toluene, benzene, diethylbenzene (Aldrich, 97% mixture of 1,2-, 1,3- 1,4- isomers) and pentane were purified by distillation over sodium benzophenone ketyl under nitrogen, then degassed via a nitrogen purge. Dimethoxyethane (DME) (Aldrich, anhydrous) was degassed via nitrogen purge and used without further purification. Pyrrole (Aldrich, 98%) was vacuum distilled over sodium, then degassed via nitrogen purge. 2,5-Dimethylpyrrole was dried with calcium sulfate and vacuum distilled. Sodium 2,5-dimethylpyrrolide ($NaC_6H_8N$) was prepared by reacting 2,5-dimethylpyrrole with an excess of sodium (40% by weight dispersion in mineral spirits) in refluxing tetrahydrofuran under nitrogen. Sodium pyrrolide was prepared by reacting pyrrole with an equivalent molar amount (1:1) of NaH (Aldrich, 60% by weight in mineral oil) or sodium (40% dispersion by weight in mineral spirits) in dimethoxyethane or tetrahydrofuran (THF) at ambient temperature under nitrogen. Triethylaluminum (TEA) (Aldrich 1.0M, hexanes and 1.9M Toluene) was used as received. Ketjen Grade B alumina ($Al_2O_3$) and Davison 952 silica ($SiO_2$) were the commercial materials used as supports for catalyst preparations. Fluorided-alumina ($F/Al_2O_3$, 15 wt% F) was prepared by the addition of a solution of $NH_4HF_2$ in methanol to Ketjen Grade B alumina. Phosphated silica ($P/SiO_2$, P/Si molar ratio = 0.1) was prepared by the addition of a 10% $H_3PO_4$/methanol solution to Davison 952 silica. The aluminophosphate ($AlPO_4$) used in the following experiments was made as described in McDaniel et al, U.S. Patent No. 4,364,855 (1982). The supports were activated by placing up to 25g into a fritted quartz tube, fluidizing with air and calcining at 700°C, except for $P/SiO_2$ at 350°C, for 3 hours. The air stream was switched to nitrogen until the support cooled to ambient temperature.

Chromium pyrrolide complexes were typically prepared from anhydrous chromium (II or III) chloride and sodium pyrrolide as follows:

A typical synthetic procedure useful to prepare chromium pyrrolide complexes was that of reacting the chromium chlorides with sodium pyrrolide ($NaC_4H_4N$, also referred to as NaPy) in refluxing tetrahydrofuran (THF). A molar reactant stoichiometry of $1CrCl_2$ and 2NaPy resulted in the isolation of a polymeric material, Product II, as the major product, and a pentanuclear complex, Product I. ($Cr_5(NC_4H_4)_{10}(OC_4H_8)_4$), as the minor product, see Equation 1. Using molar excess of NaPy resulted in the isolation of the dianionic square planar complex $\{Cr(NC_4H_4)_4\}\{Na\}_2 \cdot 2OC_4H_8$. Product III, and the octahedral complex $\{Cr(C_4H_4N)_5(OC_4H_8)\}\{Na\}_2 \cdot 4OC_4H_8$, Product IV, see Equation 2. Each of the products was isolated through precipitation (Product II) or crystallization (Products I, III, IV) from THF solutions by the addition of pentane.

$Cr_5(C_4H_4N)_{10}(C_4H_8O)_4$
Pentanuclear
Complex, (I)
Product I

THF

$1CrCl_2$ Reflux, 20 hrs.

(equation continued)

| | | | |
|---|---|---|---|
| + | ————————> | + | 1) |
| 2NaPy | Nitrogen Atm. | Polymeric Complex, (II) Product II (Major) | |

Equation 1

| | | | |
|---|---|---|---|
| | | $\{Cr(C_4H_4N)_4\}\{Na\}_2 \cdot 2OC_4H_8$ Square Planar Cr(II), Product III (Major) | |
| | THF | | |
| $CrCl_2$ | Reflux, 2 hrs. | | |
| + | ————————> | | 2) |
| NaPy | Nitrogen Atm. | $\{Cr(C_4H_4N)_5(OC_4H_8)\}\{Na\}_2 \cdot 4OC_4H_8$ | |
| (Excess) | | Octahedral Cr(III), Product IV (Minor) | |

Equation 2

Example I

To prepare the pentanuclear complex, Product I, $(Cr_5(NC_4H_4)_{10}(OC_4H_8)_4)$, and the polymeric material, Product II, chromous chloride (2.0g/16.3 mmole) was combined with sodium pyrrolide (33.7 mmole) in tetrahydrofuran and refluxed 20 hours. The reaction mixture was filtered (medium porosity frit) and the filtrate was used for fractional crystallization of both $(Cr_5(NC_4H_4)_{10}(OC_4H_8)_4)$, Product I, and the polymeric material, Product II, through the addition of pentane. The polymeric material crystallized as a blue solid followed by $(Cr_5(NC_4H_4)_{10}(OC_4H_8)_4)$ as opaque dark blue/purple crystals.

Analysis calculated for $C_{56}H_{72}N_{10}Cr_5O_4$, ProductI:C, 55.62; H, 6.00; N, 11.58%, by weight. Found: C, 55.46; H, 6.32; N, 11.15%, by weight. Analysis found for Product II: Cr, 11.5; C, 59.75; H, 7.61; N, 9.17%, by weight, but variable upon precipitation conditions. An x-ray crystal structure of Product I showed a pentanuclear complex incorporating bridging amido-pyrrolyl, terminal amido-pyrrolyl, and tetrahydrofuran ligands (Figures 1 and 2).

Example II

To prepare $\{Cr(NC_4H_4)_4\}\{Na\}_2 \cdot 2OC_4H_8$, Product III, and $\{Cr(C_4H_4N)_5(OC_4H_8)\}\{Na\}_2 \cdot 4OC_4H_8$, Product IV, chromous chloride (3.0g/24.4mmole) was combined with sodium pyrrolide (100.9mmole) in tetrahydrofuran and refluxed 2 hours, see Equation 2. The reaction mixture was filtered (medium porosity frit) and the filtrate was used for fractional crystallization of both $\{Cr(NC_4H_4)_4\}\{Na\}_2 \cdot 2OC_4H_8$, Product III, and $\{Cr(C_4H_4N)_5(OC_4H_8)\}\{Na\}_2 \cdot 4OC_4H_8$, Product IV, through the addition of pentane. Product III crystallized as translucent orange/ red crystals followed by Product IV as translucent purple crystals. While not wishing to be bound by theory, the formation of Product IV is believed to result from the presence of chromic chloride in the chromous chloride reagent (Alfa, chromium (II) chloride, anhydrous, contains 5-10% by weight $CrCl_3$) used in the preparation.

Analysis calculated for $C_{24}H_{32}N_4O_2CrNa_2$, Product III: C, 56.94: H, 6.32; N, 11.07% by weight. Found: C, 57.04; H, 6.30; N, 10.92%, by weight. Analysis calculated for $C_{40}H_{60}N_5O_5CrNa_2$, Product IV: C, 60.90; H, 7.67; N, 8.88% by weight. Found: C, 60.81; H, 7.74; N, 9.44%, by weight. An x-ray crystal structure of Product III showed a square planar complex incorporating terminal amido-pyrrolyl ligands (Figure 3). An x-ray crystal structure of Product IV showed an octahedral complex incorporating terminal amido-pyrrolyl and a tetrahydrofuran ligand (Figure 4).

Example III

The reaction product obtained from sodium pyrrolide end $CrCl_3$ was the most preferred in the preparation of an active catalyst. Pyrrole (7.0ml/100.9mmole) was mixed with NaH (4.2g of 60%, ~105 mmole) in dimethoxyethane at ambient temperature until bubbling ceased. Chromic chloride (5.33g/33.7mmole) was added to the solution at ambient temperature. The reaction mixture was refluxed under nitrogen for five hours, see Equation 3. This resulted in a dark green solution. The solution was filtered (medium porosity frit) and stripped of solvent under vacuum and pumped dry under vacuum for 12 hours. The resultant chromium pyrrolide complex was a green solid, Product V. It was used in the preparation of an active catalyst without further purification.

| | DME | | |
|---|---|---|---|
| $1CrCl_3$ | Reflux. 5 hrs. | | |
| + | ——————> | Green Solid | 3) |
| 3NaPy | Nitrogen Atm. | (V) | |

Equation 3

## Example IV

All single crystal x-ray structure analyses were performed by Crystalytics Company, Lincoln, Nebraska. Examples IV, V, and VI contain the resultant analytical and subsequently computer-generated data.

A single crystal x-ray structure was obtained for $[Cr_5(NC_4H_4)_{10}(OC_4H_8)_4]$, Product I, and shown in Figure I and Figure II. The description of the single-crystal sample and mounting used for data collection are as follows:

Color: Dark blue

Shape: Rectangular parallelepiped

Dimensions: 0.20 x 0.48 x 0.80 mm

Crystal Mount: Crystal was sealed inside a thin-walled glass capillary with epoxy under $N_2$.

Crystal Orientation: Crystal was oriented with its longest edge nearly parallel to the phi axis of the diffractometer.

Width at Half-height from W Scans: 0-38°

The space group and cell data are as follows:

Crystal System: Triclinic

Space Group and Number: PI-$C_i$ (No. 2)

Number of Computer-Centered Reflections Used in the Least-Squares Refinement of the Cell

Dimensions: 15 $2\theta>25°$ °C = 20±1°

Lattice Constants with esd's:

a = 10.803(2)Å $\alpha$ = 85.59(2)° V = 1407.9(6)Å

b = 9.825(2)Å $\beta$ = 96.23(2)° Z = 1

c = 14.212(4)Å $\gamma$ = 109.99(2)° $\tau^*$ = 0.71073Å

Molecular Weight: 1209.24 amu
Calculated Density: 1.427 g/cc
Linear Absorption Coefficient: 0.96 $mm^{-1}$

Tables I - V list the resultant parameters used to generate the molecular structure shown in Figure I and Figure II.

Table I. Atomic Coordinates for Nonhydrogen Atoms in Crystalline $[Cr_5(NC_4H_4)_{10}(OC_4H_8)_4]$ [a]

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $Cr_1$ | 0 [d] | 0 [d] | 0 [d] | 25(1) |
| $Cr_2$ | 636(1) | 2281(1) | 1500(1) | 24(1) |
| $Cr_3$ | -1179(1) | 841(1) | 3122(1) | 28(1) |
| $N_{1a}$ | -1155(3) | 935(3) | 715(2) | 25(1) |
| $C_{1a}$ | -2195(4) | 64(4) | 1231(3) | 31(1) |
| $C_{2a}$ | -3313(4) | 390(5) | 965(3) | 41(1) |
| $C_{3a}$ | -3014(4) | 1486(5) | 257(3) | 43(1) |
| $C_{4a}$ | -1728(4) | 1791(4) | 116(3) | 34(1) |
| $N_{1b}$ | 1566(3) | 1902(3) | 331(2) | 29(1) |
| $C_{1b}$ | 1753(4) | 3095(4) | -308(3) | 36(1) |
| $C_{2b}$ | 3035(5) | 3751(5) | -432(3) | 51(2) |
| $C_{3b}$ | 3736(4) | 2986(5) | 131(3) | 51(2) |
| $C_{4b}$ | 2823(4) | 1865(4) | 587(3) | 38(1) |
| $N_{1c}$ | -320(3) | 2997(3) | 2480(2) | 27(1) |
| $C_{1c}$ | 375(4) | 3732(4) | 3273(3) | 34(1) |
| $C_{2c}$ | 29(5) | 4919(4) | 3383(3) | 43(1) |
| $C_{3c}$ | -908(5) | 4967(4) | 2631(3) | 42(1) |
| $C_{4c}$ | -1105(4) | 3809(4) | 2101(3) | 32(1) |
| $N_{1d}$ | 443(3) | 350(3) | 2743(2) | 28(1) |
| $C_{1d}$ | 1600(4) | 715(4) | 3289(3) | 36(1) |
| $C_{2d}$ | 2321(4) | -133(5) | 3102(3) | 46(2) |
| $C_{3d}$ | 1567(5) | -1070(5) | 2403(3) | 46(2) |
| $C_{4d}$ | 422(4) | -763(4) | 2203(3) | 36(1) |

Table I. (continued)

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $N_{1e}$ | -1972(3) | -1122(3) | 3801(2) | 35(1) |
| $C_{1e}$ | -1344(5) | -2107(4) | 4069(3) | 41(1) |
| $C_{2e}$ | -2189(5) | -3307(4) | 4503(3) | 44(1) |
| $C_{3e}$ | -3361(5) | -3061(4) | 4531(3) | 47(1) |
| $C_{4e}$ | -3206(5) | -1731(4) | 4097(3) | 47(1) |
| $O_{1f}$ | 2351(3) | 3985(3) | 1883(2) | 32(1) |
| $C_{1f}$ | 3536(4) | 4018(4) | 2483(3) | 43(1) |
| $C_{2f}$ | 4470(6) | 5479(6) | 2336(5) | 76(2) |
| $C_{3f}$ | 3642(5) | 6408(5) | 2147(4) | 62(2) |
| $C_{4f}$ | 2396(4) | 5463(4) | 1635(3) | 40(1) |
| $O_{1g}$ | -2551(3) | 1543(3) | 3659(2) | 35(1) |
| $C_{1g}$ | -3763(4) | 1733(5) | 3232(3) | 44(1) |
| $C_{2g}$ | -4097(5) | 2625(6) | 3907(4) | 57(2) |
| $C_{3g}$ | -3524(5) | 2241(6) | 4845(3) | 57(2) |
| $C_{4g}$ | -2319(5) | 1977(6) | 4633(3) | 50(2) |

[a] The numbers in parentheses are the estimated standard deviations in the last significant digit.

[b] Atoms are labeled in agreement with Figure I.

[c] This is one-third of the trace of the orthogonalized $B_{ij}$ tensor.

[d] This is a symmetry-required value and is therefore listed without an estimated standard deviation.

Table II. Anisotropic Thermal Parameters for Nonhydrogen Atoms in Crystalline $[Cr_5(NC_4H_4)_{10}(OC_4H_8)_4]$ [a,b]

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| $Cr_1$ | 20(1) | 23(1) | 32(1) | 5(1) | 5(1) | -4(1) |
| $Cr_2$ | 23(1) | 22(1) | 27(1) | 7(1) | 3(1) | -2(1) |
| $Cr_3$ | 27(1) | 26(1) | 34(1) | 11(1) | 8(1) | 1(1) |
| $N_{1a}$ | 21(1) | 27(1) | 29(1) | 8(1) | 1(1) | -2(1) |
| $C_{1a}$ | 28(2) | 31(2) | 30(2) | 4(1) | 8(1) | -4(1) |
| $C_{2a}$ | 23(2) | 49(2) | 49(2) | 8(2) | 5(2) | -16(2) |
| $C_{3a}$ | 31(2) | 51(2) | 52(2) | 22(2) | -7(2) | -11(2) |
| $C_{4a}$ | 36(2) | 32(2) | 34(2) | 15(1) | -2(1) | -3(1) |
| $N_{1b}$ | 24(1) | 25(1) | 35(1) | 3(1) | 5(1) | -4(1) |
| $C_{1b}$ | 40(2) | 31(2) | 33(2) | 2(1) | 11(1) | -1(1) |
| $C_{2b}$ | 46(2) | 42(2) | 54(2) | -7(2) | 24(2) | -5(2) |
| $C_{3b}$ | 25(2) | 50(2) | 71(3) | -3(2) | 15(2) | -27(2) |
| $C_{4b}$ | 29(2) | 38(2) | 48(2) | 10(1) | 0(2) | -15(2) |
| $N_{1c}$ | 28(1) | 25(1) | 30(1) | 11(1) | 3(1) | -2(1) |
| $C_{1c}$ | 36(2) | 35(2) | 31(2) | 10(1) | 4(1) | -3(1) |
| $C_{2c}$ | 52(2) | 34(2) | 43(2) | 13(2) | 6(2) | -13(1) |
| $C_{3c}$ | 51(2) | 31(2) | 50(2) | 22(2) | 5(2) | -5(2) |
| $C_{4c}$ | 35(2) | 34(2) | 31(2) | 16(1) | 4(1) | 1(1) |
| $N_{1d}$ | 32(1) | 23(1) | 31(1) | 12(1) | 6(1) | 3(1) |
| $C_{1d}$ | 33(2) | 32(2) | 42(2) | 3(1) | 6(2) | -0(1) |
| $C_{2d}$ | 36(2) | 50(2) | 59(2) | 24(2) | 6(2) | 11(2) |
| $C_{3d}$ | 61(3) | 44(2) | 47(2) | 36(2) | 11(2) | 3(2) |
| $C_{4d}$ | 49(2) | 35(2) | 31(2) | 23(2) | 4(2) | 1(1) |
| $N_{1e}$ | 36(2) | 30(1) | 42(2) | 13(1) | 14(1) | 4(1) |
| $C_{1e}$ | 46(2) | 36(2) | 46(2) | 20(2) | 10(2) | 6(2) |
| $C_{2e}$ | 64(3) | 30(2) | 37(2) | 15(2) | 7(2) | 4(1) |
| $C_{3e}$ | 55(3) | 31(2) | 46(2) | -1(2) | 18(2) | -0(2) |

Table II. (continued)

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| $C_{4e}$ | 39(2) | 38(2) | 62(2) | 9(2) | 17(2) | 4(2) |
| $O_{1f}$ | 29(1) | 25(1) | 40(1) | 6(1) | -1(1) | -2(1) |
| $C_{1f}$ | 34(2) | 44(2) | 45(2) | 9(2) | -8(2) | -6(2) |
| $C_{2f}$ | 45(3) | 67(3) | 95(4) | -3(2) | -15(3) | -6(3) |
| $C_{3f}$ | 59(3) | 34(2) | 78(3) | -2(2) | -6(3) | -9(2) |
| $C_{4f}$ | 45(2) | 23(1) | 48(2) | 7(1) | 6(2) | -1(1) |
| $O_{1g}$ | 34(1) | 41(1) | 37(1) | 19(1) | 7(1) | -1(1) |
| $C_{1g}$ | 31(2) | 56(2) | 50(2) | 20(2) | 4(2) | -5(2) |
| $C_{2g}$ | 47(3) | 65(3) | 72(3) | 35(2) | 2(2) | -12(2) |
| $C_{3g}$ | 60(3) | 75(3) | 50(2) | 36(2) | 16(2) | -8(2) |
| $C_{4g}$ | 45(2) | 77(3) | 35(2) | 27(2) | 8(2) | -5(2) |

[a] The numbers in parentheses are the estimated standard deviations in the last significant digit.

[b] The form of the anisotropic thermal parameter is given in reference 8 on page 6 of the structure report.

[c] Atoms are labeled in agreement with Figure 1.

Table III. Atomic Coordinates for Hydrogen Atoms in Crystalline $[Cr_5(NC_4H_4)_{10}(OC_4H_8)_4]^a$

| Atom Type [b] | Fractional Coordinates $10^4x$ | $10^4y$ | $10^4z$ |
|---|---|---|---|
| $H_{1a}$ | -2129 | -661 | 1707 |
| $H_{2a}$ | -4154 | -55 | 1219 |
| $H_{3a}$ | -3608 | 1937 | -69 |
| $H_{4a}$ | -1267 | 2508 | -339 |
| $H_{1b}$ | 1053 | 3405 | -617 |
| $H_{2b}$ | 3405 | 4593 | -834 |
| $H_{3b}$ | 4676 | 3202 | 189 |
| $H_{4b}$ | 3031 | 1158 | 1020 |
| $H_{1c}$ | 1013 | 3445 | 3687 |
| $H_{2c}$ | 364 | 5592 | 3881 |
| $H_{3c}$ | -1331 | 5685 | 2512 |
| $H_{4c}$ | -1704 | 3580 | 1540 |
| $H_{1d}$ | 1881 | 1460 | 3743 |
| $H_{2d}$ | 3177 | -88 | 3396 |
| $H_{3d}$ | 1807 | -1790 | 2120 |
| $H_{4d}$ | -291 | -1252 | 1752 |
| $H_{1e}$ | -446 | -1976 | 3968 |
| $H_{2e}$ | -1997 | -4161 | 4742 |
| $H_{3e}$ | -4139 | -3699 | 4803 |
| $H_{4e}$ | 3878 | -1286 | 4012 |
| $H_{1fa}$ | 3351 | 3836 | 3136 |
| $H_{1fb}$ | 3882 | 3308 | 2299 |
| $H_{2fa}$ | 5068 | 5771 | 2893 |
| $H_{2fb}$ | 4965 | 5524 | 1806 |
| $H_{3fa}$ | 3462 | 6711 | 2728 |
| $H_{3fb}$ | 4068 | 7245 | 1757 |

Table III. (continued)

| Atom Type [b] | Fractional Coordinates $10^4x$ | $10^4y$ | $10^4z$ |
|---|---|---|---|
| $H_{4fa}$ | 2417 | 5653 | 964 |
| $H_{4fb}$ | 1641 | 5625 | 1839 |
| $H_{1ga}$ | -3631 | 2231 | 2623 |
| $H_{1gb}$ | -4455 | 813 | 3162 |
| $H_{2ga}$ | -5037 | 2381 | 3901 |
| $H_{2gb}$ | -3704 | 3640 | 3750 |
| $H_{3ga}$ | -4129 | 1385 | 5124 |
| $H_{3gb}$ | -3307 | 3025 | 5266 |
| $H_{4ga}$ | -2173 | 1220 | 5050 |
| $H_{4gb}$ | -1565 | 2846 | 4703 |

[a] Hydrogen atoms were included in the structure factor calculations as idealized atoms (assuming $sp^2$- or $sp^3$-hybridization of the carbon atoms and a C-H bond length of 0.96Å ) "riding" on their respective carbon atoms. The isotropic thermal parameter of each hydrogen atom was fixed at 1.2 times the equivalent isotropic thermal parameter of the carbon atom to which it is covalently bonded.

[b] Hydrogen atoms are labeled with the same numerical and literal subscripts as their carbon atoms with an additional literal subscript (a or b ) where necessary to distinguish between hydrogen atoms bonded to the same carbon.

TABLE IV. Bond Lengths Involving Nonhydrogen Atoms in Crystalline $[Cr_5(NC_4H_4)_{10}(OC_4H_8)_4]$ [a]

| Type[b] | Length, Å | Type[b] | Length, Å |
|---|---|---|---|
| $Cr_1 \cdots Cr_2$ | 3.066(1) | $O_{1f}-C_{1f}$ | 1.451(5) |
| $Cr_2 \cdots Cr_3$ | 3.121(1) | $O_{1f}-C_{4f}$ | 1.453(5) |
| | | $O_{1g}-C_{1g}$ | 1.448(6) |
| $Cr_1-N_{1a}$ | 2.153(3) | $O_{1g}-C_{4g}$ | 1.451(5) |
| $Cr_1-N_{1b}$ | 2.092(3) | | |
| $Cr_2-N_{1a}$ | 2.178(3) | $C_{1a}-C_{2a}$ | 1.360(6) |
| $Cr_2-N_{1b}$ | 2.149(3) | $C_{2a}-C_{3a}$ | 1.395(6) |
| $Cr_2-N_{1c}$ | 2.112(4) | $C_{3a}-C_{4a}$ | 1.351(6) |
| $Cr_3-N_{1c}$ | 2.172(3) | $C_{1b}-C_{2b}$ | 1.338(6) |
| $Cr_3-N_{1d}$ | 2.101(4) | $C_{2b}-C_{3b}$ | 1.393(7) |
| $Cr_3-N_{1e}$ | 2.037(3) | $C_{3b}-C_{4b}$ | 1.376(6) |
| | | $C_{1c}-C_{2c}$ | 1.365(7) |
| $Cr_2-O_{1f}$ | 2.082(2) | $C_{2c}-C_{3c}$ | 1.400(6) |
| $Cr_3-O_{1g}$ | 2.068(3) | $C_{3c}-C_{4c}$ | 1.356(6) |
| | | $C_{1d}-C_{2d}$ | 1.376(7) |
| $N_{1a}-C_{1a}$ | 1.399(4) | $C_{2d}-C_{3d}$ | 1.396(6) |
| $N_{1a}-C_{4a}$ | 1.397(5) | $C_{3d}-C_{4d}$ | 1.367(8) |
| $N_{1b}-C_{1b}$ | 1.398(5) | $C_{1e}-C_{2e}$ | 1.370(5) |
| $N_{1b}-C_{4b}$ | 1.379(6) | $C_{2e}-C_{3e}$ | 1.374(8) |
| $N_{1c}-C_{1c}$ | 1.388(4) | $C_{3e}-C_{4e}$ | 1.366(6) |

Table IV. (continued)

| Type [b] | Length, Å | Type [b] | Length, Å |
| --- | --- | --- | --- |
| $N_{1c}-C_{4c}$ | 1.394(6) | | |
| $N_{1d}-C_{1d}$ | 1.349(5) | $C_{1f}-C_{2f}$ | 1.460(6) |
| $N_{1d}-C_{4d}$ | 1.377(5) | $C_{2f}-C_{3f}$ | 1.474(9) |
| $N_{1e}-C_{1e}$ | 1.370(6) | $C_{3f}-C_{4f}$ | 1.496(6) |
| $N_{1e}-C_{4e}$ | 1.361(6) | $C_{1g}-C_{2g}$ | 1.496(8) |
| | | $C_{2g}-C_{3g}$ | 1.485(7) |
| | | $C_{3g}-C_{4g}$ | 1.476(9) |

[a] The numbers in parentheses are the estimated standard deviations in the last significant digit.
[b] Atoms are labeled in agreement with Figure 1.

Table V. Bond Angles Involving Nonhydrogen Atoms in Crystalline $[Cr_5(NC_4H_4)_{10}(OC_4H_8)_4]$ [a]

| Type[b] | Angle,deg | Type[b] | Angle,deg |
|---|---|---|---|
| $N_{1a}Cr_1N_{1b}$ | 84.8(1) | $Cr_1N_{1a}Cr_2$ | 90.2(1) |
| $N_{1a}Cr_1N_{1a'}$ [c] | 180.0(-)[d] | $Cr_1N_{1a}C_{1a}$ | 121.2(2) |
| $N_{1b}Cr_1N_{1a'}$ [c] | 95.2(1) | $Cr_2N_{1a}C_{1a}$ | 118.0(2) |
| $N_{1b}Cr_1N_{1b'}$ [c] | 180.0(-)[d] | $Cr_1N_{1a}C_{4a}$ | 113.4(2) |
| | | $Cr_2N_{1a}C_{4a}$ | 110.6(2) |
| $N_{1a}Cr_2N_{1b}$ | 82.9(1) | $C_{1a}N_{1a}C_{4a}$ | 103.5(3) |
| $N_{1a}Cr_2N_{1c}$ | 96.5(1) | $Cr_1N_{1b}Cr_2$ | 92.6(1) |
| $N_{1b}Cr_2N_{1c}$ | 168.9(1) | $Cr_1N_{1b}C_{1b}$ | 117.9(2) |
| $N_{1a}Cr_2O_{1f}$ | 162.4(1) | $Cr_2N_{1b}C_{1b}$ | 107.6(3) |
| $N_{1b}Cr_2O_{1f}$ | 89.5(1) | $Cr_1N_{1b}C_{4b}$ | 120.6(3) |
| $N_{1c}Cr_2O_{1f}$ | 87.9(1) | $Cr_2N_{1b}C_{4b}$ | 113.0(3) |
| | | $C_{1b}N_{1b}C_{4b}$ | 104.4(3) |
| $N_{1c}Cr_3N_{1d}$ | 88.1(1) | $Cr_2N_{1c}Cr_3$ | 93.5(1) |
| $N_{1c}Cr_3N_{1e}$ | 176.5(1) | $Cr_2N_{1c}C_{1c}$ | 121.4(3) |
| $N_{1d}Cr_3N_{1e}$ | 93.5(1) | $Cr_3N_{1c}C_{1c}$ | 100.0(2) |
| $N_{1c}Cr_3O_{1g}$ | 88.8(1) | $Cr_2N_{1c}C_{4c}$ | 116.1(2) |
| $N_{1d}Cr_3O_{1g}$ | 170.4(1) | $Cr_3N_{1c}C_{4c}$ | 121.5(2) |
| $N_{1e}Cr_3O_{1g}$ | 89.1(1) | $C_{1c}N_{1c}C_{4c}$ | 104.2(3) |
| | | $Cr_3N_{1d}C_{1d}$ | 121.3(3) |
| $N_{1a}C_{1a}C_{2a}$ | 110.6(3) | $Cr_3N_{1d}C_{4d}$ | 127.8(3) |
| $C_{1a}C_{2a}C_{3a}$ | 107.5(4) | $C_{1d}N_{1d}C_{4d}$ | 106.4(4) |
| $C_{2a}C_{3a}C_{4a}$ | 106.9(4) | $Cr_3N_{1e}C_{1e}$ | 126.3(3) |
| $C_{3a}C_{4a}N_{1a}$ | 111.5(3) | $Cr_3N_{1e}C_{4e}$ | 128.3(3) |
| $N_{1b}C_{1b}C_{2b}$ | 111.2(4) | $C_{1e}N_{1e}C_{4e}$ | 105.3(3) |

Table V. (continued)

| Type [b] | Angle, deg. | Type [b] | Angle, deg. |
|---|---|---|---|
| $C_{1b}C_{2b}C_{3b}$ | 107.4(4) | | |
| $C_{2b}C_{3b}C_{4b}$ | 107.0(4) | $Cr_2O_{1f}C_{1f}$ | 131.5(2) |
| $C_{3b}C_{4b}N_{1b}$ | 110.1(4) | $Cr_2O_{1f}C_{4f}$ | 118.9(2) |
| $N_{1c}C_{1c}C_{2c}$ | 110.9(4) | $C_{1f}O_{1f}C_{4f}$ | 109.1(3) |
| $C_{1c}C_{2c}C_{3c}$ | 106.8(4) | $Cr_3O_{1g}C_{1g}$ | 131.9(3) |
| $C_{2c}C_{3c}C_{4c}$ | 107.2(4) | $Cr_3O_{1g}C_{4g}$ | 118.6(3) |
| $C_{3c}C_{4c}N_{1c}$ | 110.9(3) | $C_{1g}O_{1g}C_{4g}$ | 109.5(4) |
| $N_{1d}C_{1d}C_{2d}$ | 110.3(4) | | |
| $C_{1d}C_{2d}C_{3d}$ | 106.7(4) | $O_{1f}C_{1f}C_{2f}$ | 105.0(4) |
| $C_{2d}C_{3d}C_{4d}$ | 106.6(5) | $C_{1f}C_{2f}C_{3f}$ | 104.9(4) |
| $C_{3d}C_{4d}N_{1d}$ | 109.9(3) | $C_{2f}C_{3f}C_{4f}$ | 104.4(4) |
| $N_{1e}C_{1e}C_{2e}$ | 110.0(4) | $C_{3f}C_{4f}O_{1f}$ | 105.4(4) |
| $C_{1e}C_{2e}C_{3e}$ | 107.2(4) | $O_{1g}C_{1g}C_{2g}$ | 104.8(4) |
| $C_2\ C_3\ C_4$ | 106.7(4) | $C_{1g}C_{2g}C_{3g}$ | 104.2(5) |
| $C_3\ C_4\ N_1$ | 110.8(5) | $C_{2g}C_{3g}C_{4g}$ | 104.2(4) |
| | | $C_{3g}C_{4g}O_{1g}$ | 106.1(4) |

[a] The numbers in parentheses are the estimated standard deviations in the last significant digit.

[b] Atoms are labeled in agreement with Figure 1.

[c] Atoms labeled with a prime are related to nonprimed atoms by the symmetry operation -x,-y,-z where the fractional coordinates (x,y,z) are given in Table I.

[d] This is a symmetry-required value and is therefore listed without an estimated standard deviation.

Example V

A single crystal x-ray structure was obtained for $Cr(NC_4H_4)_4$, a portion of Product III and shown in Figure III. A single crystal x-ray Structure was obtained for $[Na]_2[Cr(NC_4H_4)_4]2(OC_4H_8)$, Product III and shown in Figure IV. The description of the single-crystal sample and mounting used for the data collection

are as follows:

Color: Red-orange

Shape: Rectangular parallelepiped

Dimensions: 0.50 x 0.55 x 0.65 mm

Crystal Mount: Crystal was glued to the inside of a thin-walled glass capillary and sealed under $N_2$.

Crystal Orientation: Crystal was oriented with its longest edge nearly parallel to the phi axis of the diffractometer.

Width at Half-height from W Scans: 0.86°

The space group and cell data are as follows:

Crystal System: Monoclinic

Space Group and Number: C2/c - $C_{2h(No. 15)}$

Number of Computer-Centered Reflections Used in the Least-Squares Refinement of the Cell

Dimensions: 15 $2\theta>25°$ °C = 20±1°

Lattice Constants with esd's:

a = 9.522(2)Å $\alpha$ = 90.00° V = 2697(1)Å

b = 15.118(2)Å $\beta$ = 98.99(1)° Z = 4

c = 18.967(3)Å $\gamma$ = 90.00° $\tau$ = 0.71073Å

Molecular Weight: 506.52 amu

Calculated Density: 1.248 g/cc

Linear Absorption Coefficient: $0.47mm^{-1}$

Tables VI-X list the resultant parameters used to generate the molecular structure shown in Figure III and Figure IV.

Table VI

| Atomic Coordinates for Nonhydrogen Atoms in Crystalline $\{Na\}_2\{Cr(NC_4H_4)_4\}$ - 2 $OC_4H_8$ [a] | | | | |
|---|---|---|---|---|
| Atom Type [b] | Fractional Coordinates | | | Equivalent Isotropic Thermal Parameter, B, $Å^2$ x 10 [c] |
| | $10^4x$ | $10^4y$ | $10^4x$ | |
| Anion | | | | |
| Cr | 0[d] | 2982(1) | 2500[d] | 50(1) |
| $N_1$ | 1901(4) | 2924(2) | 3183(2) | 56(1) |
| $N_2$ | 0[d] | 4343(3) | 2500[d] | 52(1) |
| $N_3$ | 0[d] | 1612(3) | 2500[d] | 70(2) |
| $C_{11}$ | 3241(5) | 2950(3) | 3008(3) | 65(2) |
| $C_{12}$ | 4224(6) | 2768(3) | 3587(3) | 73(2) |
| $C_{13}$ | 3513(7) | 2630(4) | 4146(3) | 82(2) |
| $C_{14}$ | 2094(7) | 2734(4) | 3884(3) | 76(2) |
| $C_{21}$ | 907(5) | 4884(3) | 2926(3) | 60(1) |
| $C_{22}$ | 582(4) | 5753(3) | 2766(3) | 69(2) |
| $C_{31}$ | 390(5) | 1081(3) | 1996(4) | 94(2) |
| $C_{32}$ | 236(7) | 213(3) | 2189(5) | 133(6) |
| Cation | | | | |
| Na | 2301(2) | 6079(1) | 1783(1) | 69(1) |
| Solvent of Crystallization | | | | |
| $O_1$ | 2065(4) | 5108(2) | 830(2) | 83(1) |
| $C_{41}$ | 2759(11) | 5174(5) | 239(4) | 143(4) |
| $C_{42}$ | 2804(11) | 4319(5) | -79(4) | 148(4) |
| $C_{43}$ | 1893(10) | 3786(5) | 264(5) | 142(4) |
| $C_{44}$ | 1699(9) | 4231(4) | 902(4) | 120(3) |

[a] The numbers in parentheses are the estimated standard deviations in the last significant digit.
[b] Atoms are labeled in agreement with Figures 1 and 2.
[c] This is one-third of the trace of the orthogonalized $B_{ij}$ tensor.
[d] This is a symmetry-required value and is therefore listed without an estimated standard deviation.

Table VII

| Anisotropic Thermal Parameters for Nonhydrogen Atoms in Crystalline $\{Na\}_2\{Cr(NC_4H_4)_4\}$ - 2 $OC_4H_8$ [a,b] | | | | | | |
|---|---|---|---|---|---|---|
| Atom Type [c] | Anisotropic Thermal Parameter ($Å^2$ x 10) | | | | | |
| | $B_{11}$ | $B_{22}$ | $B_{33}$ | $B_{12}$ | $B_{13}$ | $B_{23}$ |
| Anion | | | | | | |
| Cr | 64(1) | 34(1) | 55(1) | Ø[d] | 15(1) | Ø[d] |
| $N_1$ | 69(2) | 44(2) | 56(2) | 6(1) | 12(1) | 6(1) |
| $N_2$ | 64(3) | 39(2) | 56(3) | Ø[d] | 16(2) | Ø[d] |
| $N_3$ | 65(3) | 38(2) | 1Ø7(4) | Ø[d] | 14(3) | Ø[d] |
| $C_{11}$ | 78(3) | 5Ø(2) | 7Ø(3) | -6(2) | 18(2) | 2(2) |
| $C_{12}$ | 7Ø(3) | 62(3) | 84(3) | 4(2) | 7(2) | -8(2) |
| $C_{13}$ | 1Ø3(4) | 79(3) | 58(3) | 22(3) | -8(3) | Ø(2) |
| $C_{14}$ | 86(3) | 86(3) | 58(3) | 16(3) | 16(2) | 5(2) |
| $C_{21}$ | 66(2) | 45(2) | 7Ø(3) | -2(2) | 15(2) | -6(2) |
| $C_{22}$ | 68(3) | 38(2) | 1Ø5(4) | -7(2) | 27(2) | -9(2) |
| $C_{31}$ | 65(3) | 61(3) | 152(5) | 6(2) | 9(3) | -36(3) |
| $C_{32}$ | 71(5) | 46(2) | 266(15) | 6(3) | -28(6) | -44(4) |
| Cation | | | | | | |
| Na | 7Ø(1) | 57(1) | 81(1) | -2(1) | 15(1) | -15(1) |
| Solvent of Crystallization | | | | | | |
| $O_1$ | 1Ø8(2) | 65(2) | 82(2) | -1Ø(2) | 38(2) | -16(2) |
| $C_{41}$ | 222(8) | 112(5) | 116(5) | -46(5) | 92(6) | -22(4) |
| $C_{42}$ | 192(8) | 16Ø(8) | 1Ø7(5) | 12(6) | 7Ø(5) | -32(5) |
| $C_{43}$ | 147(6) | 1Ø9(6) | 177(8) | -27(5) | 48(6) | -69(6) |
| $C_{44}$ | 177(6) | 77(4) | 124(5) | -21(4) | 76(5) | -14(3) |

[a] The numbers in parentheses are the estimated standard deviations in the last significant digit.
[b] The form of the anisotropic thermal parameter is given in reference 8 on page 6 of the structure report.
[c] Atoms are labeled in agreement with Figures 1 and 2.
[d] This is a symmetry-required value and is therefore listed without an estimated standard deviation.

Table VIII. Atomic Coordinates for Hydrogen Atoms in Crystalline $\{Na\}_2\{Cr(NC_4H_4)_4\} - 2\ OC_4H_8$ [a]

| Atom Type [b] | Fractional Coordinates $10^4x$ | $10^4y$ | $10^4z$ |
|---|---|---|---|
| | Anion | | |
| $H_{11}$ | 3456 | 3881 | 2541 |
| $H_{12}$ | 5235 | 2748 | 3688 |
| $H_{13}$ | 3922 | 2488 | 4628 |
| $H_{14}$ | 1341 | 2679 | 4164 |
| $H_{21}$ | 1665 | 4687 | 3285 |
| $H_{22}$ | 1071 | 6262 | 2985 |
| $H_{31}$ | 786 | 1274 | 1565 |
| $H_{32}$ | 483 | -381 | 1937 |
| | Solvent of Crystallization | | |
| $H_{41a}$ | 2258 | 5576 | -188 |
| $H_{41b}$ | 3718 | 5388 | 385 |
| $H_{42a}$ | 3756 | 4891 | -1 |
| $H_{42b}$ | 2464 | 4348 | -583 |
| $H_{43a}$ | 995 | 3787 | -39 |
| $H_{43b}$ | 2326 | 3228 | 377 |
| $H_{44a}$ | 2295 | 3973 | 1384 |
| $H_{44b}$ | 723 | 4191 | 969 |

[a] Hydrogen atoms were included in the structure factor calculations as idealized atoms (assuming $sp^2$- or $sp^3$-hybridization of the carbon atoms and a C-H bond length of 0.96Å) "riding" on their respective carbon atoms. The isotropic thermal parameter of each hydrogen atom was fixed at 1.2 times the equivalent isotropic thermal parameter of the carbon atom to which it is covalently bonded.

[b] Hydrogen atoms are labeled with the same numerical subscripts as the carbon atoms to which they are covalently bonded with an additional literal subscript (a or b) where necessary to distinguish between hydrogens bonded to the same carbon.

Table IX

Anion Bond Lengths and Bond Angles Involving Nonhydrogen Atoms in Crystalline $\{Na\}_2\{Cr(NC_4H_4)_4\}$ - 2 $OC_4H_8$ [a]

| Type [b] | Length, Å | Type [b] | Length, Å |
|---|---|---|---|
| Cr-$N_1$ | 2.057(3) | $C_{11}$-$C_{12}$ | 1.355(7) |
| Cr-$N_2$ | 2.056(4) | $C_{12}$-$C_{13}$ | 1.361(9) |
| Cr-$N_3$ | 2.072(5) | $C_{13}$-$C_{14}$ | 1.374(9) |
| | | $C_{21}$-$C_{22}$ | 1.372(6) |
| $N_1$-$C_{11}$ | 1.369(7) | $C_{22}$-$C_{22'}$[c] | 1.379(9) |
| $N_1$-$C_{14}$ | 1.344(6) | $C_{31}$-$C_{32}$ | 1.376(7) |
| $N_2$-$C_{21}$ | 1.360(5) | $C_{32}$-$C_{32'}$[c] | 1.327(18) |
| $N_3$-$C_{31}$ | 1.344(7) | | |
| **Type [b]** | **Angle, deg.** | **Type [b]** | **Angle, deg.** |
| $N_1CrN_2$ | 92.5(1) | $N_1C_{11}C_{12}$ | 110.5(5) |
| $N_1CrN_3$ | 87.5(1) | $C_{11}C_{12}C_{13}$ | 107.3(5) |
| $N_1CrN_{1'}$[c] | 175.1(2) | $C_{12}C_{13}C_{14}$ | 106.4(5) |
| $N_2CrN_3$ | 180.0(-)[d] | $N_1C_{14}C_{13}$ | 110.9(5) |
| | | $N_2C_{21}C_{22}$ | 110.2(4) |
| $CrN_1C_{11}$ | 127.5(3) | $C_{21}C_{22}C_{22'}$[c] | 106.8(3) |
| $CrN_1C_{14}$ | 127.1(4) | $N_3C_{31}C_{32}$ | 109.1(6) |
| $C_{11}N_1C_{14}$ | 104.9(4) | $C_{31}C_{32}C_{32'}$[c] | 107.5(5) |
| $CrN_2C_{21}$ | 127.0(2) | | |
| $C_{21}N_2C_{21'}$[c] | 106.0(5) | | |
| $CrN_3C_{31}$ | 126.7(3) | | |
| $C_{31}N_3C_{31'}$[c] | 106.7(6) | | |

[a] The numbers in parentheses are the estimated standard deviations in the last significant digit.
[b] Atoms are labeled in agreement with Figure 1.
[c] Atoms labeled with a prime(') are related to nonprimed atoms by the symmetry operation -x,y,$\frac{1}{2}$-z.

Table X

| Bond Lengths and Angles Involving the Nonhydrogen Atoms of the Cation and Solvent of Crystallization in $\{Na\}_2\{Cr(NC_4H_4)_4\}$ - 2 $OC_4H_8$ [a] | | | |
|---|---|---|---|
| Type [b] | Length, Å | Type [b] | Length, Å |
| Na-$O_1$ | 2.313(4) | $O_1$-$C_{41}$ | 1.390(10) |
| | | $O_1$-$C_{44}$ | 1.382(7) |
| Na•••$N_{1''}$ [c] | 2.888(4) | | |
| Na•••$N_{3''}$ [c] | 2.830(4) | $C_{41}$-$C_{42}$ | 1.43(1) |
| | | $C_{42}$-$C_{43}$ | 1.42(1) |
| | | $C_{43}$-$C_{44}$ | 1.42(1) |
| Type [b] | Angle, deg. | Type [b] | Angle, deg. |
| $O_1NaN_{1''}$ [c] | 128.6(3) | $C_{41}O_1C_{44}$ | 107.9(5) |
| $O_1NaN_{3''}$ [c] | 121.8(3) | | |
| $N_{1''}NaN_{3''}$ [c] | 59.9(3) | $O_1C_{41}C_{42}$ | 109.0(7) |
| | | $C_{41}C_{42}C_{43}$ | 105.0(8) |
| $NaO_1C_{41}$ | 125.7(4) | $C_{42}C_{43}C_{44}$ | 107.0(7) |
| $NaO_1C_{44}$ | 121.8(4) | $O_1C_{44}C_{43}$ | 107.6(7) |

[a] The numbers in parentheses are the estimated standard deviations in the last significant digit.
[b] Atoms are labeled in agreement with Figure 2.
[c] Atoms labeled with a double prime('') are related to nonprimed atoms by the symmetry operation $\frac{1}{2}$-x,$\frac{1}{2}$+y,$\frac{1}{2}$-z

Example VI

Single crystal x-ray structures were obtained for $[Cr(NC_4H_4)_5(OC_4H_8)]$, shown in Figure V, + $[Cr(NC_4H_4)_5(OC_4H_8)][Na]_2$ -4($OC_4H_8$), Product IV, + shown in Figure VI. The description of the single-crystal sample and mounting used for data collection are as follows:

Color: Purple

Shape: Rectangular parallelepiped

Dimensions: 0.50 x 0.55 x 0.63 mm

Crystal Mount: Crystal was glued to the inside of a thin-walled glass capillary and sealed under $N_2$.

Crystal Orientation: Crystal was oriented with its longest edge nearly parallel to the phi axis of the diffractometer.

Width at Half-height from W Scans: 0.42°

The space group and cell data are as follows:

Crystal System: Monoclinic

Space Group and Number: $P2_1$-$C_2^2$(No. 4)

Number of Computer-Centered Reflections Used in the Least-Squares Refinement of the Cell Dimensions:

15 $2\theta>20°$ °C = 20±1°

Lattice Constants with esd's:

$$a = 10.042(2)Å \quad \alpha = 90.00° \quad V = 2162(1)Å$$
$$b = 17.242(4)Å \quad \beta = 106.54(2)° \quad Z = 2$$
$$c = 13.025(3)Å \quad \gamma = 90.00° \quad \tau = 0.71073Å$$

Molecular Weight = 788.93 amu

Calculated Density: 1.212 g/cc

Linear Absorption Coefficient: 0.32 $mm^{-1}$

Tables XI - XV list the resultant parameters used to generate the molecular structure shown in Figure V and Figure VI.

Table XI. Atomic Coordinates for Nonhydrogen Atoms in Crystalline $[Cr(NC_4H_4)_5(OC_4H_8)][Na]_2 \cdot 4\ OC_4H_8$ [a]

| Atom Type [b] | Fractional Coordinates $10^4x$ | $10^4y$ | $10^4z$ | Equivalent Isotropic Thermal Parameter, B, $Å^2 \times 10$ [c] |
|---|---|---|---|---|
| | | Anion | | |
| Cr | 198(1) | 1477 | 2531(1) | 32(1) |
| $N_{1a}$ | 1694(5) | 2026(3) | 2028(4) | 40(2) |
| $C_{1a}$ | 1749(7) | 2782(4) | 1742(6) | 48(2) |
| $C_{2a}$ | 2929(8) | 2926(5) | 1420(7) | 66(3) |
| $C_{3a}$ | 3661(7) | 2236(5) | 1554(6) | 62(3) |
| $C_{4a}$ | 2899(6) | 1695(5) | 1913(5) | 52(2) |
| $N_{1b}$ | 1651(5) | 1087(3) | 3885(4) | 40(2) |
| $C_{1b}$ | 1463(8) | 560(4) | 4575(5) | 48(2) |
| $C_{2b}$ | 2572(9) | 518(6) | 5423(8) | 82(4) |
| $C_{3b}$ | 3554(8) | 1064(6) | 5275(6) | 70(3) |
| $C_{4b}$ | 2952(6) | 1382(5) | 4340(5) | 48(2) |
| $N_{1c}$ | -1326(5) | 1888(3) | 1250(4) | 38(2) |
| $C_{1c}$ | -1200(8) | 2172(4) | 266(6) | 51(2) |
| $C_{2c}$ | -2458(8) | 2270(5) | -476(6) | 58(3) |
| $C_{3c}$ | -3435(8) | 2038(6) | 56(7) | 75(3) |
| $C_{4c}$ | -2710(7) | 1826(5) | 1091(6) | 56(3) |
| $N_{1d}$ | -32(5) | 2455(4) | 3445(5) | 43(2) |
| $C_{1d}$ | 504(7) | 2562(5) | 4505(6) | 49(2) |
| $C_{2d}$ | 107(9) | 3278(5) | 4774(8) | 72(3) |
| $C_{3d}$ | -698(8) | 3629(5) | 3832(6) | 59(3) |
| $C_{4d}$ | -769(7) | 3108(4) | 3055(6) | 52(2) |
| $N_{1e}$ | 315(5) | 505(4) | 1690(4) | 40(2) |
| $C_{1e}$ | -574(8) | 277(5) | 704(6) | 55(3) |
| $C_{2e}$ | -197(10) | -432(5) | 403(7) | 67(3) |
| $C_{3e}$ | 990(10) | -662(6) | 1256(8) | 79(4) |
| $C_{4e}$ | 1265(8) | -92(4) | 2016(7) | 51(3) |

Table XI. (continued)

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $O_{1f}$ | -1356(4) | 926(3) | 3083(4) | 43(1) |
| $C_{1f}$ | -2047(7) | 1244(5) | 3800(6) | 57(3) |
| $C_{2f}$ | -3263(10) | 713(6) | 3706(9) | 98(5) |
| $C_{3f}$ | -2833(11) | -21(6) | 3402(8) | 93(4) |
| $C_{4f}$ | -1903(8) | 171(5) | 2724(7) | 64(3) |
| | | Cation 1 | | |
| $Na_1$ | 2254(3) | 3336(2) | 3737(3) | 75(1) |
| | | Cation 2 | | |
| $Na_2$ | 1430(3) | 974(2) | 126(2) | 62(1) |
| | | Solvent Molecules of Crystallization | | |
| $O_{1g}$ | 4576(6) | 3329(4) | 4706(5) | 83(2) |
| $C_{1g}$ | 5748(9) | 3100(10) | 4433(9) | 125(6) |
| $C_{2g}$ | 6723(12) | 2831(11) | 5281(9) | 145(7) |
| $C_{3g}$ | 6503(15) | 3272(11) | 6146(11) | 204(8) |
| $C_{4g}$ | 5037(14) | 3498(11) | 5737(10) | 170(8) |
| $O_{1h}$ | 2342(7) | 4602(4) | 3279(6) | 97(3) |
| $C_{1h}$ | 1316(11) | 5151(7) | 2894(10) | 112(5) |
| $C_{2h}$ | 2017(16) | 5830(9) | 2541(11) | 153(7) |
| $C_{3h}$ | 3180(12) | 5561(10) | 2425(10) | 131(6) |
| $C_{4h}$ | 3551(13) | 4848(7) | 3070(11) | 115(6) |
| $O_{1i}$ | 1391(7) | 1752(4) | -1377(4) | 80(2) |
| $C_{1i}$ | 2235(19) | 1594(11) | -1998(13) | 160(8) |
| $C_{2i}$ | 2716(17) | 2287(14) | -2337(15) | 165(10) |
| $C_{3i}$ | 1991(28) | 2906(11) | -1934(14) | 204(12) |
| $C_{4i}$ | 1010(16) | 2533(7) | -1523(9) | 128(6) |

Table XI. (continued)

| Atom Type [b] | Fractional Coordinates $10^4x$ | $10^4y$ | $10^4z$ | Equivalent Isotropic Thermal Parameter, B, $Å^2$ x 10 [c] |
|---|---|---|---|---|
| $O_{1j}$ | 3037(5) | 155(4) | -264(5) | 72(2) |
| $C_{1j}$ | 4389(10) | 48(7) | 427(9) | 113(5) |
| $C_{2j}$ | 4998(16) | -571(10) | -23(16) | 174(8) |
| $C_{3j}$ | 4001(11) | -840(8) | -1006(10) | 127(6) |
| $C_{4j}$ | 2728(11) | -493(7) | -974(8) | 92(4) |

[a] The numbers in parentheses are the estimated standard deviations in the last significant digit.
[b] Atoms are labeled in agreement with Figures 1 and 2.
[c] This is one-third of the trace of the orthogonalized $B_{ij}$ tensor.

Table XII. Anisotropic Thermal Parameters for Nonhydrogen Atoms in Crystalline $[Cr(NC_4H_4)_5(OC_4H_8)][Na]_2 \cdot 4\ OC_4H_8$ [a,b]

| Atom Type [c] | Anisotropic Thermal Parameter ($Å^2$ x 10) | | | | | |
|---|---|---|---|---|---|---|
| | $B_{11}$ | $B_{22}$ | $B_{33}$ | $B_{12}$ | $B_{13}$ | $B_{23}$ |
| | | | Anion | | | |
| Cr | 29(1) | 31(1) | 38(1) | 1(1) | 12(1) | 1(1) |
| $N_{1a}$ | 33(2) | 44(3) | 44(3) | -1(2) | 11(2) | 5(2) |
| $C_{1a}$ | 48(4) | 37(3) | 59(4) | -0(3) | 15(3) | 3(3) |
| $C_{2a}$ | 55(4) | 61(5) | 90(5) | -19(4) | 34(4) | 13(4) |
| $C_{3a}$ | 37(3) | 82(6) | 76(5) | -9(3) | 33(3) | 2(4) |
| $C_{4a}$ | 40(3) | 64(5) | 52(4) | 4(3) | 16(3) | -5(3) |
| $N_{1b}$ | 36(2) | 44(3) | 36(3) | 7(2) | 5(2) | 12(2) |
| $C_{1b}$ | 52(4) | 51(4) | 40(3) | -1(3) | 9(3) | 10(3) |
| $C_{2b}$ | 73(5) | 85(6) | 83(6) | 2(5) | 13(4) | 44(5) |
| $C_{3b}$ | 51(4) | 88(6) | 54(4) | 0(4) | -13(3) | 12(4) |
| $C_{4b}$ | 41(3) | 55(4) | 45(3) | 0(3) | 5(2) | 4(4) |
| $N_{1c}$ | 33(2) | 41(3) | 39(3) | 4(2) | 9(2) | 1(2) |
| $C_{1c}$ | 52(4) | 51(4) | 51(4) | 6(3) | 16(3) | 5(3) |
| $C_{2c}$ | 64(5) | 62(5) | 37(4) | -1(4) | -4(3) | 4(4) |
| $C_{3c}$ | 32(3) | 92(6) | 89(6) | 4(4) | -3(4) | 29(5) |
| $C_{4c}$ | 42(3) | 78(5) | 48(4) | -1(3) | 9(3) | 14(4) |
| $N_{1d}$ | 31(2) | 44(3) | 56(3) | 4(2) | 13(2) | -1(3) |
| $C_{1d}$ | 44(3) | 60(5) | 39(4) | -5(3) | 8(3) | -11(3) |
| $C_{2d}$ | 63(4) | 70(6) | 84(6) | -11(4) | 20(4) | -47(5) |
| $C_{3d}$ | 69(4) | 43(4) | 73(5) | 9(3) | 32(4) | -14(4) |
| $C_{4d}$ | 42(3) | 53(4) | 63(4) | 8(3) | 17(3) | 3(4) |
| $N_{1e}$ | 47(3) | 36(3) | 39(3) | -3(2) | 17(2) | -7(2) |
| $C_{1e}$ | 59(4) | 49(4) | 53(4) | -15(3) | 11(3) | -1(4) |
| $C_{2e}$ | 92(5) | 48(4) | 69(5) | -20(4) | 36(4) | -26(4) |
| $C_{3e}$ | 91(6) | 45(5) | 106(7) | 4(4) | 37(5) | -13(5) |
| $C_{4e}$ | 62(4) | 23(3) | 69(5) | 7(3) | 20(4) | -7(3) |

**Table XII. (continued)**

| Atom Type [c] | Anisotropic Thermal Parameter ($Å^2$ x 10) $B_{11}$ | $B_{22}$ | $B_{33}$ | $B_{12}$ | $B_{13}$ | $B_{23}$ |
|---|---|---|---|---|---|---|
| $O_{1f}$ | 40(2) | 42(2) | 51(2) | -4(2) | 20(2) | 2(2) |
| $C_{1f}$ | 61(4) | 64(5) | 60(4) | -2(3) | 39(3) | 4(4) |
| $C_{2f}$ | 81(6) | 95(7) | 144(8) | -24(5) | 74(6) | 1(6) |
| $C_{3f}$ | 109(7) | 80(6) | 117(7) | -26(5) | 75(6) | -3(6) |
| $C_{4f}$ | 61(4) | 53(4) | 85(5) | -27(4) | 30(4) | -16(4) |
| | | | Cation 1 | | | |
| $Na_1$ | 57(2) | 71(2) | 95(2) | -13(1) | 21(2) | -2(2) |
| | | | Cation 2 | | | |
| $Na_2$ | 68(2) | 69(2) | 56(2) | -2(1) | 30(1) | -3(2) |
| | | | Solvent Molecules of Crystallization | | | |
| $O_{1g}$ | 58(3) | 95(4) | 92(4) | -8(3) | 15(3) | -2(4) |
| $C_{1g}$ | 54(5) | 215(14) | 108(8) | 0(7) | 29(5) | -7(9) |
| $C_{2g}$ | 96(7) | 226(15) | 121(9) | 52(9) | 43(7) | 51(10) |
| $C_{3g}$ | 129(10) | 277(19) | 148(11) | 52(12) | -56(9) | -134(13) |
| $C_{4g}$ | 134(10) | 250(18) | 128(10) | 44(11) | 39(9) | -89(11) |
| $O_{1h}$ | 71(4) | 68(4) | 152(6) | -8(3) | 32(4) | -3(4) |
| $C_{1h}$ | 92(7) | 95(8) | 144(9) | -2(6) | 28(7) | -3(7) |
| $C_{2h}$ | 212(14) | 108(9) | 140(10) | 36(10) | 50(10) | 66(9) |
| $C_{3h}$ | 99(8) | 175(14) | 101(8) | -6(9) | -2(6) | 32(9) |
| $C_{4h}$ | 99(8) | 79(7) | 168(11) | -13(6) | 38(8) | 29(8) |
| $O_{1i}$ | 98(4) | 82(4) | 73(3) | 8(3) | 47(3) | 13(3) |
| $C_{1i}$ | 230(15) | 128(11) | 168(12) | 8(11) | 131(12) | 74(10) |
| $C_{2i}$ | 112(10) | 222(21) | 156(15) | 1(12) | 28(10) | 23(16) |
| $C_{3i}$ | 370(26) | 124(12) | 135(12) | -93(15) | 99(15) | 34(10) |
| $C_{4i}$ | 223(13) | 81(7) | 106(8) | 32(8) | 91(9) | 31(6) |

**Table** XII. **(continued)**

| Atom Type [c] | Anisotropic Thermal Parameter (Å$^2$ x 10) $B_{11}$ | $B_{22}$ | $B_{33}$ | $B_{12}$ | $B_{13}$ | $B_{23}$ |
|---|---|---|---|---|---|---|
| $O_{1j}$ | 59(3) | 64(3) | 94(4) | 5(3) | 22(3) | -21(3) |
| $C_{1j}$ | 88(7) | 101(8) | 133(9) | 19(6) | 2(6) | -58(7) |
| $C_{2j}$ | 94(8) | 190(14) | 205(13) | 73(10) | -11(9) | -90(13) |
| $C_{3j}$ | 83(7) | 130(10) | 160(10) | 16(7) | 20(7) | -86(9) |
| $C_{4j}$ | 82(6) | 104(8) | 92(7) | -7(6) | 29(5) | -41(6) |

[a] The numbers in parentheses are the estimated standard deviations in the last significant digit.

[b] The form of the anisotropic thermal parameter is given in reference 8 on page 6 of the structure report.

[c] Atoms are labeled in agreement with Figures 1 and 2.

Table XIII. Atomic Coordinates for Hydrogen Atoms in Crystalline $[Cr(NC_4H_4)_5(OC_4H_8)][Na]_2 - 4\ OC_4H_8$ [a]

| Atom Type [b] | Fractional Coordinates $10^4x$ | $10^4y$ | $10^4z$ |
|---|---|---|---|
| | | Anion | |
| $H_{1a}$ | 1061 | 3165 | 1756 |
| $H_{2a}$ | 3182 | 3406 | 1151 |
| $H_{3a}$ | 4547 | 2153 | 1428 |
| $H_{4a}$ | 3162 | 1162 | 2059 |
| $H_{1b}$ | 637 | 254 | 4479 |
| $H_{2b}$ | 2692 | 174 | 6022 |
| $H_{3b}$ | 4453 | 1179 | 5753 |
| $H_{4b}$ | 3373 | 1775 | 4016 |
| $H_{1c}$ | -326 | 2281 | 132 |
| $H_{2c}$ | -2637 | 2453 | -1199 |
| $H_{3c}$ | -4426 | 2031 | -243 |
| $H_{4c}$ | -3137 | 1655 | 1623 |
| $H_{1d}$ | 1070 | 2197 | 4997 |
| $H_{2d}$ | 349 | 3499 | 5480 |
| $H_{3d}$ | -1115 | 4135 | 3762 |
| $H_{4d}$ | -1278 | 3184 | 2317 |
| $H_{1e}$ | -1346 | 578 | 293 |
| $H_{2e}$ | -630 | -712 | -243 |
| $H_{3e}$ | 1503 | -1135 | 1285 |
| $H_{4e}$ | 1999 | -107 | 2676 |
| $H_{1fa}$ | -1447 | 1250 | 4520 |
| $H_{1fb}$ | -2359 | 1762 | 3588 |
| $H_{2fa}$ | -4069 | 899 | 3170 |
| $H_{2fb}$ | -3468 | 674 | 4380 |
| $H_{3fa}$ | -2341 | -312 | 4022 |
| $H_{3fb}$ | -3620 | -314 | 2996 |

Table XIII. (continued)

| Atom Type [b] | Fractional Coordinates $10^4x$ | $10^4y$ | $10^4z$ |
|---|---|---|---|
| $H_{4fa}$ | -2417 | 184 | 1980 |
| $H_{4fb}$ | -1165 | -201 | 2831 |
| | Solvent of Crystallization | | |
| $H_{1ga}$ | 6103 | 3536 | 4135 |
| $H_{1gb}$ | 5503 | 2694 | 3909 |
| $H_{2ga}$ | 6629 | 2283 | 5371 |
| $H_{2gb}$ | 7629 | 2940 | 5209 |
| $H_{3ga}$ | 6644 | 2947 | 6766 |
| $H_{3gb}$ | 7102 | 3717 | 6322 |
| $H_{4ga}$ | 4960 | 4045 | 5839 |
| $H_{4gb}$ | 4493 | 3223 | 6118 |
| $H_{1ha}$ | 596 | 4950 | 2301 |
| $H_{1hb}$ | 921 | 5310 | 3451 |
| $H_{2ha}$ | 2205 | 6231 | 3073 |
| $H_{2hb}$ | 1449 | 6034 | 1874 |
| $H_{3ha}$ | 3066 | 5447 | 1684 |
| $H_{3hb}$ | 3908 | 5936 | 2669 |
| $H_{4ha}$ | 4260 | 4953 | 3725 |
| $H_{4hb}$ | 3874 | 4459 | 2671 |
| $H_{1ia}$ | 3007 | 1289 | -1594 |
| $H_{1ib}$ | 1721 | 1306 | -2615 |
| $H_{2ia}$ | 3703 | 2328 | -2031 |
| $H_{2ib}$ | 2496 | 2303 | -3103 |
| $H_{3ia}$ | 1541 | 3249 | -2509 |
| $H_{3ib}$ | 2638 | 3195 | -1381 |
| $H_{4ia}$ | 101 | 2580 | -2020 |
| $H_{4ib}$ | 1010 | 2761 | -851 |

Table XIII. (continued)

| Atom Type [b] | Fractional Coordinates $10^4x$ | $10^4y$ | $10^4z$ |
|---|---|---|---|
| $H_{1ja}$ | 4929 | 513 | 470 |
| $H_{1jb}$ | 4341 | -91 | 1129 |
| $H_{2ja}$ | 5823 | -388 | -178 |
| $H_{2jb}$ | 5232 | -992 | 479 |
| $H_{3ja}$ | 3930 | -1396 | -1018 |
| $H_{3jb}$ | 4261 | -668 | -1623 |
| $H_{4ja}$ | 2185 | -862 | -715 |
| $H_{4jb}$ | 2215 | -324 | -1678 |

[a] Hydrogen atoms were included in the structure factor calculations as idealized atoms (assuming $sp^2$- or $sp^3$-hybridization of the carbon atoms and a C-H bond length of 0.96Å ) "riding" on their respective carbon atoms. The isotropic thermal parameter of each hydrogen atom was fixed at 1.2 times the equivalent isotropic thermal parameter of the carbon atom to which it is covalently bonded.

[b] Hydrogen atoms are labeled with the same numerical and literal subscripts as their carbon atoms with an additional literal subscript (a, or b ) where necessary to distinguish between hydrogen atoms bonded to the same carbon.

Table XIV. Bond Lengths Involving Nonhydrogen Atoms in Crystalline $[Cr(NC_4H_4)_5(OC_4H_8)][Na]_2 \cdot 4\ OC_4H_8$ [a]

| Type [b] | Length, Å | Type [b] | Length, Å |
|---|---|---|---|
| $Cr-N_{1a}$ | 2.035(6) | $Na_1-O_{1g}$ | 2.314(6) |
| $Cr-N_{1b}$ | 2.056(5) | $Na_1-O_{1h}$ | 2.271(8) |
| $Cr-N_{1c}$ | 2.044(5) | | |
| $Cr-N_{1d}$ | 2.114(6) | $Na_2-O_{1i}$ | 2.365(7) |
| $Cr-N_{1e}$ | 2.024(6) | $Na_2-O_{1j}$ | 2.307(7) |
| $Cr-O_{1f}$ | 2.120(5) | $C_{1g}-C_{2g}$ | 1.33(2) |
| | | $C_{2g}-C_{3g}$ | 1.43(2) |
| $N_{1a}-C_{1a}$ | 1.36(1) | $C_{3g}-C_{4g}$ | 1.47(2) |
| $N_{1a}-C_{4a}$ | 1.38(1) | $C_{1h}-C_{2h}$ | 1.51(2) |
| $N_{1b}-C_{1b}$ | 1.33(1) | $C_{2h}-C_{3h}$ | 1.30(2) |
| $N_{1b}-C_{4b}$ | 1.37(1) | $C_{3h}-C_{4h}$ | 1.48(2) |
| $N_{1c}-C_{1c}$ | 1.41(1) | $C_{1i}-C_{2i}$ | 1.41(3) |
| $N_{1c}-C_{4c}$ | 1.35(1) | $C_{2i}-C_{3i}$ | 1.47(3) |
| $N_{1d}-C_{1d}$ | 1.34(1) | $C_{3i}-C_{4i}$ | 1.40(3) |
| $N_{1d}-C_{4d}$ | 1.36(1) | $C_{1j}-C_{2j}$ | 1.44(2) |
| $N_{1e}-C_{1e}$ | 1.40(1) | $C_{2j}-C_{3j}$ | 1.46(2) |
| $N_{1e}-C_{4e}$ | 1.39(1) | $C_{3j}-C_{4j}$ | 1.42(2) |
| $O_{1f}-C_{1f}$ | 1.42(1) | $O_{1g}-C_{1g}$ | 1.38(1) |
| $O_{1f}-C_{4f}$ | 1.44(1) | $O_{1g}-C_{4g}$ | 1.32(1) |
| | | $O_{1h}-C_{1h}$ | 1.38(1) |

Table XIV. (continued)

| Type [b] | Length, Å | Type [b] | Length, Å |
|---|---|---|---|
| $C_{1a}-C_{2a}$ | 1.39(1) | $O_{1h}-C_{4h}$ | 1.39(2) |
| $C_{2a}-C_{3a}$ | 1.38(1) | $O_{1i}-C_{1i}$ | 1.36(2) |
| $C_{3a}-C_{4a}$ | 1.37(1) | $O_{1i}-C_{4i}$ | 1.40(1) |
| $C_{1b}-C_{2b}$ | 1.33(1) | $O_{1j}-C_{1j}$ | 1.41(1) |
| $C_{2b}-C_{3b}$ | 1.42(1) | $O_{1j}-C_{4j}$ | 1.43(1) |
| $C_{3b}-C_{4b}$ | 1.31(1) | | |
| $C_{1c}-C_{2c}$ | 1.37(1) | $Na_1-C_{1a}$ | 2.678(8) |
| $C_{2c}-C_{3c}$ | 1.41(1) | $Na_1-N_{1d}$ | 2.688(7) |
| $C_{3c}-C_{4c}$ | 1.39(1) | $Na_1-C_{1d}$ | 2.621(9) |
| $C_{1d}-C_{2d}$ | 1.37(1) | | |
| $C_{2d}-C_{3d}$ | 1.40(1) | | |
| $C_{3d}-C_{4d}$ | 1.34(1) | $Na_2-C_{4a}$ | 2.681(7) |
| $C_{1e}-C_{2e}$ | 1.37(1) | $Na_2-C_{1e}$ | 2.630(9) |
| $C_{2e}-C_{3e}$ | 1.43(1) | | |
| $C_{3e}-C_{4e}$ | 1.37(1) | | |
| $C_{1f}-C_{2f}$ | 1.50(1) | | |
| $C_{2f}-C_{3f}$ | 1.43(2) | | |
| $C_{3f}-C_{4f}$ | 1.49(2) | | |

[a] The numbers in parentheses are the estimated standard deviations in the last significant digit.

[b] Atoms are labeled in agreement with Figure 1.

Table XV. Bond Angles Involving Nonhydrogen Atoms in Crystalline $[Cr(NC_4H_4)_5(OC_4H_8)][Na]_2\text{-}4\ OC_4H_8$ [a]

| Type [b] | Angle,deg | Type [b] | Angle,deg |
|---|---|---|---|
| $N_{1a}CrN_{1b}$ | 91.2(2) | $O_{1g}Na_1O_{1h}$ | 92.3(3) |
| $N_{1a}CrN_{1c}$ | 91.4(2) | $O_{1g}Na_1C_{1a}$ | 114.3(3) |
| $N_{1a}CrN_{1d}$ | 91.1(2) | $O_{1g}Na_1N_{1d}$ | 139.6(3) |
| $N_{1a}CrN_{1e}$ | 92.8(2) | $O_{1g}Na_1C_{1d}$ | 118.0(3) |
| $N_{1a}CrO_{1f}$ | 178.7(2) | $O_{1h}Na_1C_{1a}$ | 95.6(3) |
| $N_{1b}CrN_{1c}$ | 176.2(2) | $O_{1h}Na_1N_{1d}$ | 127.0(2) |
| $N_{1b}CrN_{1d}$ | 86.7(2) | $O_{1h}Na_1C_{1d}$ | 132.1(3) |
| $N_{1b}CrN_{1e}$ | 93.3(2) | $C_{1a}Na_1N_{1d}$ | 75.1(2) |
| $N_{1b}CrO_{1f}$ | 88.5(2) | $C_{1a}Na_1C_{1d}$ | 103.1(3) |
| $N_{1c}CrN_{1d}$ | 90.4(2) | $N_{1d}Na_1C_{1d}$ | 29.3(2) |
| $N_{1c}CrN_{1e}$ | 89.4(2) | | |
| $N_{1c}CrO_{1f}$ | 88.8(2) | $O_{1i}Na_2O_{1j}$ | 90.7(3) |
| $N_{1d}CrN_{1e}$ | 176.1(2) | $O_{1i}Na_2C_{4a}$ | 109.3(3) |
| $N_{1d}CrO_{1f}$ | 87.6(2) | $O_{1i}Na_2C_{1e}$ | 131.5(2) |
| $N_{1e}CrO_{1f}$ | 88.5(2) | $O_{1j}Na_2C_{4a}$ | 103.2(2) |
| | | $O_{1j}Na_2C_{1e}$ | 115.1(3) |
| $CrN_{1a}C_{1a}$ | 128.7(5) | $C_{4a}Na_2C_{1e}$ | 103.9(3) |
| $CrN_{1a}C_{4a}$ | 126.3(5) | | |
| $CrN_{1b}C_{1b}$ | 127.0(4) | $Na_1O_{1g}C_{1g}$ | 131.4(6) |
| $CrN_{1b}C_{4b}$ | 127.3(5) | $Na_1O_{1g}C_{4g}$ | 124.0(8) |
| $CrN_{1c}C_{1c}$ | 128.5(5) | $Na_1O_{1h}C_{1h}$ | 132.2(7) |

**Table XV. (continued)**

| Type [b] | Angle,deg | Type [b] | Angle,deg |
|---|---|---|---|
| $CrN_{1c}C_{4c}$ | 126.7(5) | $Na_1O_{1h}C_{4h}$ | 116.6(6) |
| $CrN_{1d}C_{1d}$ | 127.7(5) | $Na_2O_{1i}C_{1i}$ | 120.9(8) |
| $CrN_{1d}C_{4d}$ | 125.7(5) | $Na_2O_{1i}C_{4i}$ | 126.8(7) |
| $CrN_{1e}C_{1e}$ | 127.7(5) | $Na_2O_{1j}C_{1j}$ | 123.1(6) |
| $CrN_{1e}C_{4e}$ | 126.2(4) | $Na_2O_{1j}C_{4j}$ | 125.8(5) |
| | | | |
| $CrO_{1f}C_{1f}$ | 126.4(4) | $C_{1g}O_{1g}C_{4g}$ | 104.3(8) |
| $CrO_{1f}C_{4f}$ | 123.1(5) | $C_{1h}O_{1h}C_{4h}$ | 108.9(9) |
| | | $C_{1i}O_{1i}C_{4i}$ | 107.8(11) |
| $C_{1a}N_{1a}C_{4a}$ | 105.0(6) | $C_{1j}O_{1j}C_{4j}$ | 107.7(7) |
| $C_{1b}N_{1b}C_{4b}$ | 105.2(5) | | |
| $C_{1c}N_{1c}C_{4c}$ | 104.0(5) | $O_{1g}C_{1g}C_{2g}$ | 111(1) |
| $C_{1d}N_{1d}C_{4d}$ | 106.6(6) | $C_{1g}C_{2g}C_{3g}$ | 103(1) |
| $C_{1e}N_{1e}C_{4e}$ | 106.0(6) | $C_{2g}C_{3g}C_{4g}$ | 103(1) |
| | | $C_{3g}C_{4g}O_{1g}$ | 110(1) |
| $C_{1f}O_{1f}C_{4f}$ | 110.5(6) | $O_{1h}C_{1h}C_{2h}$ | 106(1) |
| | | $C_{1h}C_{2h}C_{3h}$ | 106(1) |
| $N_{1a}C_{1a}C_{2a}$ | 111.1(7) | $C_{2h}C_{3h}C_{4h}$ | 109(1) |
| $C_{1a}C_{2a}C_{3a}$ | 106.1(8) | $C_{3h}C_{4h}O_{1h}$ | 106(1) |
| $C_{2a}C_{3a}C_{4a}$ | 107.5(7) | $O_{1i}C_{1i}C_{2i}$ | 110(2) |
| $C_{3a}C_{4a}N_{1a}$ | 110.3(7) | $C_{1i}C_{2i}C_{3i}$ | 105(2) |

**Table XV. (continued)**

| Type [b] | Angle,deg | Type [b] | Angle,deg |
|---|---|---|---|
| $N_{1b}C_{1b}C_{2b}$ | 110.6(7) | $C_{2i}C_{3i}C_{4i}$ | 106(2) |
| $C_{1b}C_{2b}C_{3b}$ | 107.6(8) | $C_{3i}C_{4i}O_{1i}$ | 107(1) |
| $C_{2b}C_{3b}C_{4b}$ | 104.4(7) | $O_{1j}C_{1j}C_{2j}$ | 106(1) |
| $C_{3b}C_{4b}N_{1b}$ | 112.2(7) | $C_{1j}C_{2j}C_{3j}$ | 109(1) |
| $N_{1c}C_{1c}C_{2c}$ | 112.4(7) | $C_{2j}C_{3j}C_{4j}$ | 104(1) |
| $C_{1c}C_{2c}C_{3c}$ | 104.5(7) | $C_{3j}C_{4j}O_{1j}$ | 108(1) |
| $C_{2c}C_{3c}C_{4c}$ | 107.8(7) | | |
| $C_{3c}C_{4c}N_{1c}$ | 111.2(7) | $Na_1C_{1a}N_{1a}$ | 95.0(4) |
| $N_{1d}C_{1d}C_{2d}$ | 109.0(7) | $Na_1C_{1a}C_{2a}$ | 106.7(5) |
| $C_{1d}C_{2d}C_{3d}$ | 107.6(8) | $Na_1N_{1d}Cr$ | 107.7(3) |
| $C_{2d}C_{3d}C_{4d}$ | 105.4(8) | $Na_1N_{1d}C_{1d}$ | 72.6(4) |
| $C_{3d}C_{4d}N_{1d}$ | 111.5(7) | $Na_1N_{1d}C_{4d}$ | 86.4(4) |
| $N_{1e}C_{1e}C_{2e}$ | 111.0(7) | $Na_1C_{1d}N_{1d}$ | 78.1(4) |
| $C_{1e}C_{2e}C_{3e}$ | 105.2(7) | $Na_1C_{1d}C_{2d}$ | 85.1(6) |
| $C_{2e}C_{3e}C_{4e}$ | 108.4(8) | | |
| $C_{3e}C_{4e}N_{1e}$ | 109.5(7) | $Na_2C_{4a}N_{1a}$ | 90.2(3) |
| | | $Na_2C_{4a}C_{3a}$ | 104.0(5) |
| $O_{1f}C_{1f}C_{2f}$ | 104.4(7) | $Na_2C_{1e}N_{1e}$ | 78.1(4) |
| $C_{1f}C_{2f}C_{3f}$ | 105.0(9) | $Na_2C_{1e}C_{2e}$ | 91.7(6) |
| $C_{2f}C_{3f}C_{4f}$ | 104.9(9) | | |
| $C_{3f}C_{4f}O_{1f}$ | 104.7(7) | | |

**Table XV. (continued)**

[a] The numbers in parentheses are the estimated standard deviations in the last significant digit.
[b] Atoms are labeled in agreement with Figure 1.

Example VII

The product obtained from the reaction of sodium 2,5-dimethylpyrrolide and $CrCl_2$ used in the preparation of an active catalyst was a light blue solid, Product VI. 2,5-Dimethylpyrrole (5.0ml/49.1mmole) was mixed with excess sodium (40% dispersion in mineral spirits) in tetrahydrofuran (125ml) at ambient temperature. The mixture was refluxed 12 hours under nitrogen then filtered to remove excess sodium. The sodium 2,5-dimethylpyrrolide was used in-situ and combined with chromous chloride (3.03g/24.7mmole) at ambient temperature. The reaction mixture was refluxed under nitrogen for 48 hours. The gray-green solution was filtered (medium porosity frit) at ambient temperature and stripped of solvent under vacuum, then pumped dry under vacuum for 12 hours resulting in a gray/green solid. This grey/green solid was then washed with pentane resulting in a light blue solid, Product VI, which was collected by filtration. Product VI was used in the preparation of an active catalyst without further purification.

Example VIII

All polymerization runs were carried out in a two liter reactor under slurry (particle form) conditions. The diluent was isobutane and the reactor temperature was 90 °C. Reactor pressure held at 3900 kPa (550 psig) during the polymerization, with ethylene being fed on demand.

The actual charging of the reactor was accomplished by the following method. After purging the reactor at 100 °C with a stream of nitrogen for at least 15 minutes, the reactor temperature was lowered to 90 °C and a preweighed amount of supported chromium pyrrolide catalyst was charged against a slight countercurrent of nitrogen. One liter of isobutane was then charged to the reactor and finally the reactor pressurized with ethylene.

The ethylene consumed was determined using a precalibrated ethylene flow meter. Samples of the liquid product mixture were taken after 30 minute run time without depressurizing the reactor. This was done by filling to 1480-2170 kPa (200-300 psig) a steel sampling cylinder adapted to the reactor with a dip tube fitted with a fritted tip extending into the bottom of the reactor vessel. Samples taken this way were analyzed by gas chromatography and gas chromatography-mass spectrometry. Selectivities were normalized to 100%. Solid products were obtained by venting the reactor to atmosphere, separating by decantation of the liquids from the solid material. The solids were then dried at 100 °C in a vacuum oven and weighed. The yield of solid product was obtained by weighing the combined solid and catalyst residues and subtracting from this the preweighed catalyst charge. The yield of volatile products was obtained by subtracting the yield of solid products from the grams of ethylene consumed as recorded by the flow meter.

Activity typically ranged from 300-1500 g product/g catalyst/hour calculated for 30 minute run time, as shown in Table XVI. The product obtained typically was represented by 97-99.5% by weight liquids and 0.5-3% by weight polymer (wax). The liquid fraction was typically 85% hexenes, 11% decenes, 2% tetradecenes, based on the total weight of the liquid fraction. The balance of the liquid product mixture was a trace level distribution of olefins typically totalling about 1-2% by weight of the product mixture, see Table XVII.

Active catalysts were prepared from the chromium pyrrolide complexes as follows. All toluene and/or pentane rinses used 15 to 30mls of liquid.

0.158g of Product V (prepared in THF solvent), which was heated to 80 °C for 4 hours under nitrogen flush to remove residual THF, was slurried with 15ml toluene at ambient temperature. 9.0ml of a 1M TEA in hexanes solution was added to the solution and stirred for 24 hours. The formation of a brown solution and the complete dissolution of Product V resulted immediately upon TEA addition. $AlPO_4$ (P/Al mole ratio = 0.4) (2.00g) was added to the solution and stirred for an additional 24 hours. The supported catalyst was filtered from the solution as a brown solid, rinsed twice with toluene, and then twice with pentane. 0.3143g

of the catalyst was charged directly to the reactor for polymerization. 1.0ml of a 0.5% TEA in heptanes solution was charged to the reactor after the catalyst charge but before the isobutane (reactor solvent) charge to purge feedstock poisons.

Run 2: 0.081g of Product V (prepared in THF solvent), which was heated to 80 °C for 4 hours under nitrogen flush to remove residual THF, was slurried with 15ml diethylbenzene at ambient temperature. 2.0ml of a 1M TEA in hexanes solution was added to the solution and stirred for 24 hours. The formation of a brown solution and the complete dissolution of Product V resulted immediately upon TEA addition. $AlPO_4$-(P/Al mole ratio = 0.4) (1.50g) was added to the solution and stirred for an additional 1 hour. The supported catalyst was filtered from the solution as a brown solid, rinsed twice with dimethylbenzene, and then twice with pentane. 0.4333g of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 3: 0.093g of Product V (prepared in DME solvent) was slurried with 15ml toluene at ambient temperature. 5.0ml of a 1M TEA in hexanes solution was added to the solution and stirred for 24 hours. The formation of a brown solution and the complete dissolution of Product V resulted immediately upon TEA addition. $AlPO_4$ (P/Al mole ratio = 0.4) (1.0g) was added to the solution and stirred for an additional 24 hours. The supported catalyst was filtered from the solution as a brown solid, rinsed twice with toluene, and then twice with pentane. 0.1564g of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

0.080g of Product I (prepared in THF solvent) was slurried with 15ml toluene at ambient temperature. 6.0ml of a 1M TEA in hexanes solution was added and the solution stirred for 16 hours. The formation of a brown solution and the complete dissolution of Product I resulted immediately upon TEA addition. $AlPO_4$-(P/Al mole ratio = 0.4) (1.50g) was added to the solution and stirred for an additional 16 hours. The supported catalyst was filtered from the solution as a brown solid, rinsed twice with toluene, and then twice pentane 1.1988g of the catalyst was charged directly to the reactor for polymerization.

Run 5: 0.079g of Product II (prepared in THF solvent) was slurried with 15ml toluene at ambient temperature. 2.0ml of a 1.9M TEA in toluene solution was added to the solution and stirred for 8 hours. The formation of a brown solution and the complete dissolution of Product II resulted immediately upon TEA addition. $AlPO_4$ (P/Al mole ratio = 0.4) (0.50g) was added to the solution and stirred for an additional 16 hours. The supported catalyst was filtered from the solution as a brown solid, rinsed twice with toluene, and then tiwce with pentane. 0.4829g of the catalyst was charged directly to the reactor for polymerization.

Run 6: 0.071g of Product V (prepared in THF solvent), which was heated to 80 °C for 4 hours under nitrogen flush to remove residual THF, was slurried with 15ml toluene at ambient temperature. 2.0ml of a 1M TEA in hexanes solution was added to the solution and stirred for 1 hour. The formation of a brown solution and the complete dissolution of Product V resulted immediately upon TEA addition. $SiO_2$ (2.52g) was added to the solution and stirred for an additional 2 minutes. The supported catalyst was filtered from the solution as a brown solid, rinsed twice with toluene, and then twice with pentane. All of the catalyst was charged directly to the reactor for polymerization.

Run 7: 0.103g of Product II (prepared in THF solvent) was slurried with 15ml toluene at ambient temperature. 1.0ml of a 1.9M TEA in toluene solution was added to the solution and stirred for 10 minutes. The formation of a brown solution and the complete dissolution of Product II resulted immediately upon TEA addition. A1203 (2.27g) was added to the solution and stirred for an additional 2 minutes. The supported catalsyt was filtered from the solution as a brown solid. rinsed twice with toluene, and then twice with pentane. 1.2926g of the catalyst was charged directly to the reactor for polymerization.

Run 8: 0.120g of Product I (prepared in THF solvent) was slurried with 15ml toluene at ambient temperature. 2.0ml of a 1M TEA in hexanes solution was added to the solution and stirred for 2 days. The formation of a brown solution and the complete dissolution of Product I resulted immediately upon TEA addition. $SiO_2$ (1.0g) was added to the solution and stirred for an additional 3 weeks. The supported catalyst was filtered from the solution as a brown solid, rinsed twice with toluene, and then twice with pentane. All of the catalyst was charged directly to the reactor for polymerization.

Run 9: 0.106g of Product III (prepared in THF solvent) was slurried with 15ml toluene at ambient temperature. 2.5ml of a 1.9M TEA in toluene solution was added to the solution and stirred for 2 hours. The formation of a brown solution and the complete dissolution of Product III resulted immediately upon TEA addition. $AlPO_4$ (P/Al mole ratio = 0.4) (0.65g) was added to the solution and stirred for an additional 2 hours. The supported catalyst was filtered from the solution as a brown solid, rinsed twice with toluene, and then twice with pentane. All of the catalyst was charged directly to the reactor for polymerization. 1.5ml of a 1.0% TEA in pentane solution was charged to the reactor after the catalyst charge but before the isobutane

(reactor solvent) charge to purge feedstock poisons.

Run 10: 0.030g of Product V (prepared in THF solvent) which was heated to 80°C for 4 hours under nitrogen flush to remove residual THF was slurried with 15ml toluene at ambient temperature. 3.0ml of a 1M TEA in hexanes solution was added to the solution and stirred for 16 hours. The formation of a brown solution and the complete dissolution of Product V resulted immediately upon TEA addition. $AlPO_4$ (P/Al = .9) (2.0g) was added to the solution and stirred for an additional 16 hours. The supported catalyst was filtered from the solution as a brown solid, rinsed twice with toluene, and then twice with pentane. 0.322/g of catalyst was charged directly to the reactor for polymerization.

Run 11: 0.067g of Product V (prepared in THF solvent) was slurried with 15ml pentane at ambient temperature. 4.0ml of a 1M TEA in hexanes solution was added to the solution and stirred for 24 hours. The formation of a brown solution and the complete dissolution of Product V resulted immediately upon TEA addition. $AlPO_4$ (P/Al mole ratio = 0.4) (1.0g) was added to the solution and stirred for an additional 24 hours. The supported catalyst was filtered from the solution as a brown solid, rinsed twice with pentane. All of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 12: 0.073g of Product V (prepared in THF solvent), which was heated to 80°C for 4 hours under nitrogen flush to remove residual THF, was slurried with 15ml toluene at ambient temperature. 6.0ml of a 1M TEA in hexanes solution was added and the solution stirred for 24 hours. The formation of a brown solution and the complete dissolution of Product V resulted immediately upon TEA addition. $P/SiO_2$ (7.0g) was added to the solution and stirred for an additional 24 hours which nearly decolorized it. The supported catalyst was filtered from the solution as a brown solid, rinsed twice with toluene, and then twice with pentane. 2.85g of catalyst was charged directly to the reactor for polymerization.

Run 13: 0.125g of Product II was slurried with 15ml diethylbenzene at ambient temperature. 9.0ml of a 1M TEA in hexanes solution was added to the solution and stirred for 8 hours. The formation of a brown solution and the complete dissolution of Product II resulted immediately upon TEA addition. $P/Al_2O_3$ (2.0g) was added to the solution and stirred for an additional 12 hours. The supported catalyst was filtered from the solution as a brown solid, rinsed twice with toluene, and then twice with pentane. 0.5477g of catalyst was charged directly to the reactor for polymerization.

Run 14: 0.125g of Product VI was slurried with 15ml toluene at ambient temperature. 1.5ml of a 1M TEA in hexanes solution was added and the solution stirred for 10 minutes. The formation of a red/brown solution and the complete dissolution of Product VI resulted immediately upon TEA addition. $SiO_2$ (2.0g) was added to the solution and stirred for an additional 1 minute which nearly decolorized it. The supported silica catalyst was filtered from the solution as a red/brown solid. rinsed twice with toluene, and then twice with pentane. All of the catalyst was charged directly to the reactor for polymerization.

Run 15: 0.30g of Product V (prepared in DME solvent) was dissolved with 15ml of dimethoxyethane forming a green solution. This solution was then mixed with 0.6g of $AlPO_4$ ($P/Al_4$ mole ratio = 0.4) (2.00g) and the mixture was stirred 1 hour. The green supported material was filtered from the solution, rinsed with dimethoxyethane and dried with a nitrogen purge at 90°C. This material was then stirred with 15ml of toluene and 3ml of triethylaluminum (Aldrich 1.0M, hexanes) for an additional 3 hours. The brown supported catalyst was collected by filtration, rinsed with pentane, and dried under vacuum. 0.4609g of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 16: 0.058g of Product V (prepared in THF solvent) which was heated to 80°C for 4 hours under nitrogen flush to remove residual THF was slurried with 15ml benzene at ambient temperature. 4.0ml of a 1M TEA in hexanes solution was added and the solution stirred for 2 hours. The formation of a brown solution and the complete dissolution of Product V resulted immediately upon TEA addition. $AlPO_4$ (P/Al mole ratio = 0.4) (1.0g) was added to the solution and stirred for 1 hour. The supported catalyst was filtered from the solution as a brown solid, rinsed twice with benzene, and then twice with pentane. All of the catalyst was charged directly to the reactor for polymerization. 3.0ml of a 0.5% TEA in heptanes solution was charged to the reactor after the catalyst charge but before the isobutane (reactor solvent) charge to purge feedstock poisons.

Run 17: 0.1610g of Product I was charged directly to the reactor at 90°C. The reactor was charged with 1 liter isobutane and pressurized to 3900 kPa (550 psig) with ethylene. No ethylene consumption was detected, therefore 450 kPa (50 psig) of dihydrogen was charged to the reactor which did not initiate ethylene consumption. Ethylene consumption was initiated after 2.0ml of 1M TEA in hexanes solution was charged.

Run 18: 0.3528g of Product VI was charged directly to the reactor at 90 °C. The reactor was charged with 1 liter isobutane and pressurized to 3900 kPa (550 psig) with ethylene. No ethylene consumption was detected, therefore 2.0ml of a 1M TEA in hexanes solution was charged which did initiate ethylene consumption.

Run 19: 0.3482g of Product VI was charged directly to the reactor at 90 °C. The reactor was also charged with 2.0ml of a 1M TEA in hexanes solution prior to a 1 liter isobutane charge. The reactor was then pressurized to 3900 kPa (550 psig) with ethylene. No ethylene consumption was detected, therefore 310 kPa (30 psi) of dihydrogen was charged to the reactor which initiated ethylene consumption.

Table XVI

| Run[a] | Catalyst | Support[b] | Products | Activity[c] |
|---|---|---|---|---|
| 1 | (V)/TEA/Toluene | $AlPO_4$ | 98.4% liquids, 1.6% solids | 1030 |
| 2 | (V)/TEA/Diethylbenzene | $AlPO_4$ | 99.4% liquids, 0.6% solids | 730 |
| 3 | (V)/TEA/Toluene | $AlPO_4$ | 99.4% liquids, 0.6% solids | 1450[d] |
| 4 | (I)/TEA/Toluene | $AlPO_4$ | 98.6% liquids, 1.4% solids | 360 |
| 5 | (II)/TEA/Toluene | $AlPO_4$ | 98.2% liquids, 1.8% solids | 580 |
| 6 | (V)/TEA/Toluene | $SiO_2$ | 89.0% liquids, 11.0% solids | 80 |
| 7 | (II)/TEA/Toluene | $Al_2O_3$ | 55.8% liquids, 44.2% solids | 50 |
| 8 | (I)/TEA/Toluene | $SiO_2$ | 93.3% liquids, 6.7% solids | 400 |
| 9 | (III)/TEA/Toluene | $AlPO_4$ | 99.8% liquids. 0.2% solids | 100 |
| 10 | (V)/TEA/Toluene | $AlPO_4$(.9) | 96.8% liquids, 3.2% solids | 930 |
| 11 | (V)/TEA/Pentane | $AlPO_4$ | (trace) liquids, (trace) solids | unreactive |
| 12 | (V)/TEA/Toluene | $P/SiO_2$ | 98.1% liquids, 1.9% solids | 90 |
| 13 | (II)/TEA/Diethylbenzene | $F/Al_2O_3$ | 88.0% liquids, 12.0% solids | 300 |
| 14 | (VI)/TEA/Toluene | $SiO_2$ | 94.3% liquids, 5.7% solids | 40 |
| 15 | (V)/DME | $AlPO_4$ | 98.0% liquids, 2.0% solids | 550 |

**Table XVI Cont.**

| Run[a] | Catalyst | Support[b] | Products | Activity[c] |
|---|---|---|---|---|
| 16 | (V)/TEA/Benzene | $AlPO_4$ | 99.1% liquids, 0.9% solids | 500 |
| 17 | (I)/TEA | unsupported | 98.3% liquids, 1.7% solids | 340 |
| 18 | (VI)/TEA | unsupported | 99.4% liquids, 0.6% solids | 180 |
| 19 | (VI)/TEA | unsupported | 98.1% liquids, 1.9% solids | 230 |

[a] All runs were made at 90°C, isobutane solvent, 3900 KPa (550 psi) total pressure.

[b] P/Al mole ratio = 0.4; except Run 10 whereby P/Al mole ratio = 0.9

[c] Grams product/grams chromium/hour based on 30 min. run times.

[d] Believed to be lower than actual due to experimental error; actual value believed to be near 2000.

Table XVII

| Run | C4 | 1-hexene | C6 | C8 | C10 | C12 | C14 | C16-C28 |
|---|---|---|---|---|---|---|---|---|
| 1 | 0.05 | 81.92 | 7.76 | 0.49 | 9.12 | 0.09 | 0.52 | .05 |
| 2 | 0.10 | 78.80 | 7.49 | 0.58 | 11.36 | 0.10 | 1.01 | .56 |
| 3 | 0.06 | 82.19 | 7.68 | 0.45 | 8.85 | 0.08 | 0.58 | .11 |
| 5 | 0.10 | 83.40 | 7.08 | 0.62 | 8.08 | 0.05 | 0.42 | .25 |
| 6 | 0.55 | 78.70 | 5.52 | 1.84 | 11.24 | 0.42 | 1.26 | .47 |
| 16 | 0.06 | 72.85 | 13.61 | 0.31 | 12.06 | 0.09 | 0.93 | .09 |
| 19 | 6.03 | 71.66 | 6.09 | 3.61 | 9.42 | 1.17 | 1.41 | .61 |

The data in Table XVI show that the inventive chromium compounds can be used either supported (Runs 1-16) or unsupported (Runs 17-19) to polymerize and/or trimerize olefins. Furthermore, conditions can be varied to increase the amount of trimer product (Runs 1-5 and 9) or to have a higher yield of solid, or polymer, product (Runs 6, 7, and 13). Runs 1 and 3 demonstrate that high activities are attainable.

Example IX

All polymerization runs were carried out as described in Example VIII.

Density was determined in grams per cubic centimeter (g/cc) on a compression molded sample, cooled at 15°C per hour, and conditioned for 40 hours at room temperature in accordance with ASTM D1505 and ASTM D1928, condition C. The high load melt index (HLMI) was determined in accordance with ASTM D1238 at 190°C with a 21,600 gram weight. Melt index (MI) was determined according to ASTM D1238 at 190°C with a 2,160 gram weight.

Run 20: 0.100g of Product V (prepared in THF solvent), which was heated to 80 C for 4 hours under nitrogen flush to remove residual THF, was slurried with 15ml toluene at ambient temperature. 7.0ml of a 1M TEA in hexanes solution was added and the solution stirred for 24 hours. The formation of a brown solution and the complete dissolution of Product V resulted immediately upon TEA addition. An $AlPO_4$ (P/Al mole ratio = 0.4) (2.0g) was added to the solution and stirred for 2 hours. The supported catalyst was filtered from the solution as a brown solid, rinsed twice with toluene, and then twice with pentane. 0.225g of this catalyst was mixed with 0.078g of a tergel-supported chromium catalyst as two free-flowing powders. The tergel-supported chromium catalyst was prepared in accordance with procedures in U.S. Patent 3,887,494. 0.2576g of this catalyst mixture was charged directly to the polymerization reactor. 3.0ml of a 0.5% TEA in heptanes solution was charged to the reactor after the catalyst charge, but before the isobutane (reactor solvent) charge, to purge feedstock poisons. The reactor was pressurized to 3900 kPa (550 psig) with ethylene. After a 60.8 minute run time, 190g of polymer was produced. The polymer had a MI = 6.5, HLMI = 366.2, and a denstiy equal to 0.0132 g/cc. The catalyst activity was 730 g polymer/g catalyst/hr based on a 60.8 minute run time (run temperature 90°C, 3900 kPa (550 psig) total pressure).

Run 21: 0.100g of Product V (prepared in THF solvent), which was heated to 80°C for 4 hours under nitrogen flush to remove residual THF, was slurried with 15ml toluene at ambient temperature. 7.0ml of a 1M TEA in hexanes solution was added and the solution stirred for 24 hours. The formation of a brown solution and the complete dissolution of Product V resulted immediately upon TEA addition. An $AlPO_4$ support (P/Al mole ratio = 0.4) (2.0g) was added to the solution and stirred for 2 hours. The supported catalyst was filtered from the solution as a brown solid, rinsed twice with toluene, and then twice with pentane. 0.2314g of this catalyst was mixed with 0.0184g of a titanium-containing catalyst as two free-flowing powders. The titanium-containing catalyst was prepared in accordance with procedures in U.S. Patents 4,325,837 and U.S. 4,326,988 wherein ethylaluminumdichloride was the aluminum alkyl used. 0.2385g of this catalyst mixture was charged directly to the polymerization reactor. 3.0ml of a 0.5 percent TEA in heptanes solution was charged to the reactor after the catalyst charge, but before the isobutane (1 liter, the reactor solvent) charge, to purge feedstock poisons. 450 kPa 50 psig of dihydrogen ($H_2$) was then charged to the reactor, followed by pressurizing to 3900 kPa (550 psig) with ethylene. After a 31.0 minute run time, 82g of polymer was produced. The polymer had a MI = 0.011, HLMI = 0.63, and a density equal to 0.9429 g/cc. The catalyst activity was 670g polymer/g catalyst/hr based on a 31.0 minute run time (run temp. 90°C, 3900 kPa (550 psig) total pressure).

While this invention has been described in detail for the purpose of illustration, it is not to be construed as limited thereby but is intended to cover all changes and modifications within the spirit and scope thereof.

## Claims

1. A process for polymerizing olefins in the presence of a polymerization catalyst and a cocatalyst, characterized by said cocatalyst having been prepared by reacting a mixture of a chromium salt, a metal amide and an ether.

2. The process of claim 1 wherein said polymerization catalyst is a supported chromium catalyst.

3. The process of claim 1 wherein said polymerization catalyst is a titanium-containing catalyst.

4. The process of any of the preceding claims wherein said cocatalyst is a chromium compound selected from $Cr_5(C_4H_4N)_{10}(C_4H_8O)_4$; $Cr(C_4H_4N)_4$; $[Cr(C_4H_4N)_4][Na]_2x2(OC_4H_8)$; $[Cr(C_4H_4N)_5(OC_4H_8)]$; $[Cr(C_4H_4N)_5(OC_4H_8)][Na]_2x4(OC_4H_8)$; and mixtures thereof.

5. The process of any of the preceding claims wherein said cocatalyst is supported on an inorganic oxide.

6. The process of any of the preceding claims wherein said olefin has from 2 to 30 carbon atoms.

7. The process of claim 6 wherein said olefin is selected from ethylene, propylene, 1-butene, 1-hexene, and mixtures thereof.

8. The process of claim 7 wherein said olefin is predominantly ethylene.

9. The process of any of the preceding claims wherein said polymerization is carried out at a temperature within the range of 66 to 110 °C.

10. The process of any of the preceding claims wherein hydrogen is present during said polymerization.

11. The process of any of the preceding claims being carried out as slurry polymerization.

12. The process of any of the preceding claims wherein said olefin is ethylene and the recovered polymer has a density within the range of 0.9 to 0. 93 g/ml.

**Patentansprüche**

1. Verfahren zur Polymerisation von Olefinen in Anwesenheit eines Polymerisationskatalysators und eines Cokatalysators, dadurch gekennzeichnet, daß der Cokatalysator durch Umsetzung eines Gemisches eines Chromsalzes, eines Metallamids und eines Ethers hergestellt worden ist.

2. Verfahren nach Anspruch 1, wobei es sich beim Polymerisationskatalysator um einen Träger-Chromkatalysator handelt.

3. Verfahren nach Anspruch 1, wobei es sich beim Polymerisationskatalysator um einen Titan enthaltenden Katalysator handelt.

4. Verfahren nach einem der vorstehenden Ansprüche, wobei es sich beim Cokatalysator um eine Chromverbindung, ausgewählt unter $Cr_5(C_4H_4N)_{10}(C_4H_8O)_4$, $Cr(C_4H_4N)_4$, $[Cr(C_4H_4N)_4][Na]_2x2(OC_4H_8)$, $[Cr(C_4H_4N)_5(OC_4H_8)]$, $[Cr(C_4H_4N)_5(OC_4H_8)][Na]_2x4(OC_4H_8)$ und Gemische davon, handelt.

5. Verfahren nach einem der vorstehenden Ansprüche, wobei der Cokatalysator auf ein anorganisches Oxid aufgetragen ist.

6. Verfahren nach einem der vorstehenden Ansprüche, wobei das Olefin 2 bis 30 Kohlenstoffatome aufweist.

7. Verfahren nach Anspruch 6, wobei das Olefin unter Ethylen, Propylen, 1-Buten, 1-Hexen und Gemischen davon ausgewählt ist.

8. Verfahren nach Anspruch 7, wobei es sich beim Olefin vorwiegend um Ethylen handelt.

9. Verfahren nach einem der vorstehenden Ansprüche, wobei die Polymerisation bei einer Temperatur im Bereich von 66 bis 110 °C durchgeführt wird.

10. Verfahren nach einem der vorstehenden Ansprüche, wobei während der Polymerisation Wasserstoff anwesend ist.

11. Verfahren nach einem der vorstehenden Ansprüche, durchgeführt als Aufschlämmungspolymerisation.

12. Verfahren nach einem der vorstehenden Ansprüche, wobei es sich beim Olefin um Ethylen handelt und das gewonnene Polymere eine Dichte im Bereich von 0,9 bis 0,93 g/ml aufweist.

**Revendications**

1. Un procédé pour la polymérisation d'oléfines en présence d'un catalyseur de polymérisation et d'un co-catalyseur, caractérisé en ce que ledit co-catalyseur a été préparé en faisant réagir un mélange d'un sel de chrome, d'un amide de métal et d'un éther.

2. Le procédé selon la revendication 1, dans lequel ledit catalyseur de polymérisation est un catalyseur de chrome fixé sur un support.

3. Le procédé selon la revendication 1, dans lequel ledit catalyseur de polymérisation est un catalyseur contenant du titane.

4. Le procédé selon l'une quelconque des revendications précédentes, dans lequel ledit co-catalyseur est un composé de chrome choisi parmi $Cr_5(C_4H_4N)_{10}(C_4H_8O)_4$ ; $Cr(C_4H_4N)_4$; $[Cr(C_4H_4N)_4][Na]_2x2-(OC_4H_8)$ ; $[Cr(C_4H_4N)_5(OC_4H_8)]$ ; $[Cr(C_4H_4N)_5](OC_4H_8)][Na]_2x4(OC_4H_8)$ et leurs mélanges.

5. Le procédé selon l'une quelconque des revendications précédentes, dans lequel ledit co-catalyseur est fixé sur un oxyde non organique.

6. Le procédé selon l'une quelconque des revendications précédentes, dans lequel ladite oléfine renferme de 2 à 30 atomes de carbone.

7. Le procédé selon la revendication 6, dans lequel ladite oléfine est choisie parmi l'éthylène, le propylène, le 1-butène, le 1-hexène et leurs mélanges.

8. Le procédé selon la revendication 7, dans lequel ladite oléfine est de façon prédominante de l'éthylène.

9. Le procédé selon l'une quelconque des revendications précédentes, dans lequel ladite polymérisation est mise en oeuvre à une température comprise dans la gamme de 66 à 110 °C.

10. Le procédé selon l'une quelconque des revendications précédentes, dans lequel de l'hydrogène est présent pendant ladite polymérisation.

11. Le procédé selon l'une quelconque des revendications précédentes qui est mis en oeuvre selon une polymérisation en suspension.

12. Le procédé selon l'une quelconque des revendications précédentes, dans lequel ladite oléfine est l'éthylène et le polymère récupéré a une densité comprise dans la gamme de 0,9 à 0,93 g/ml.

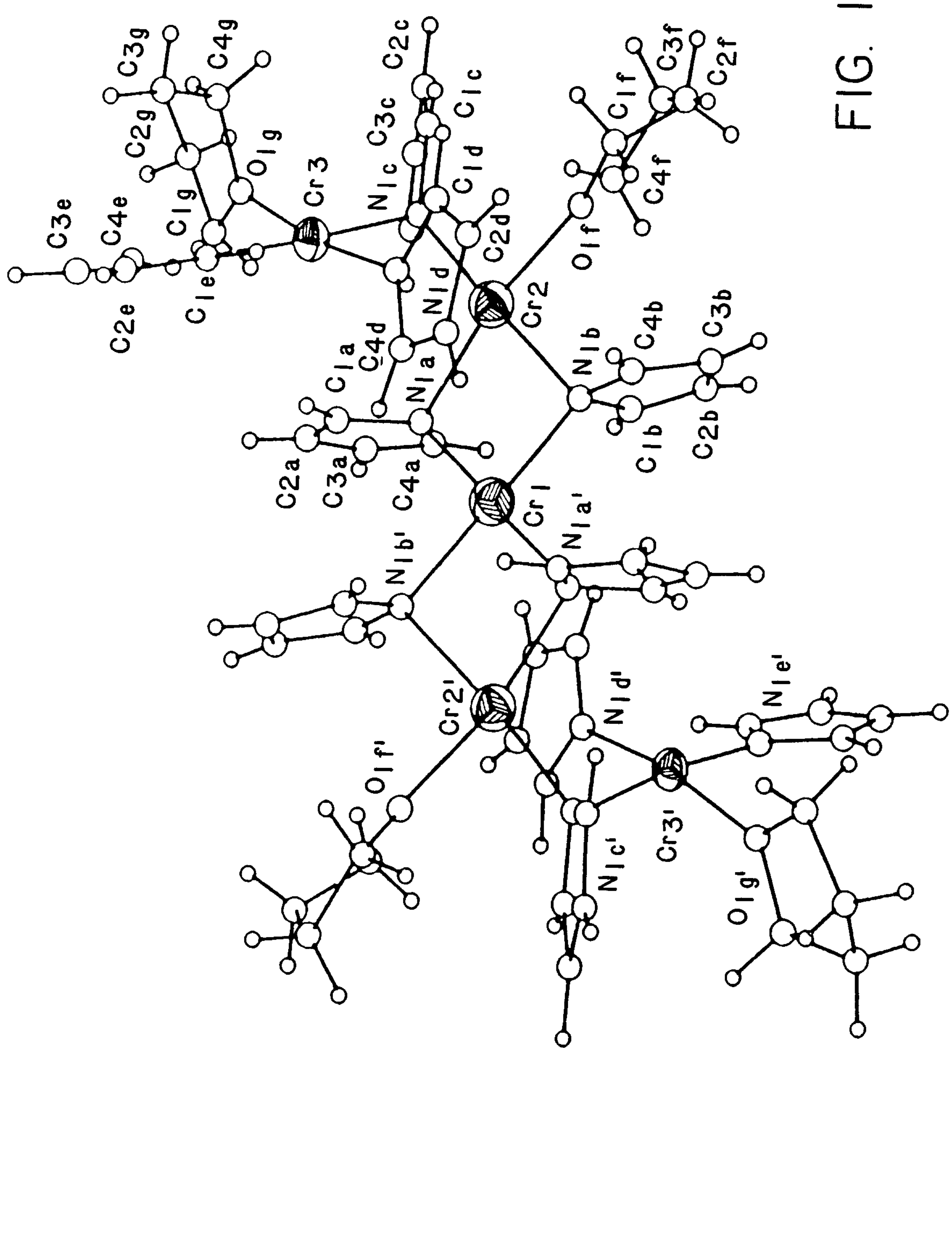

FIG. 1

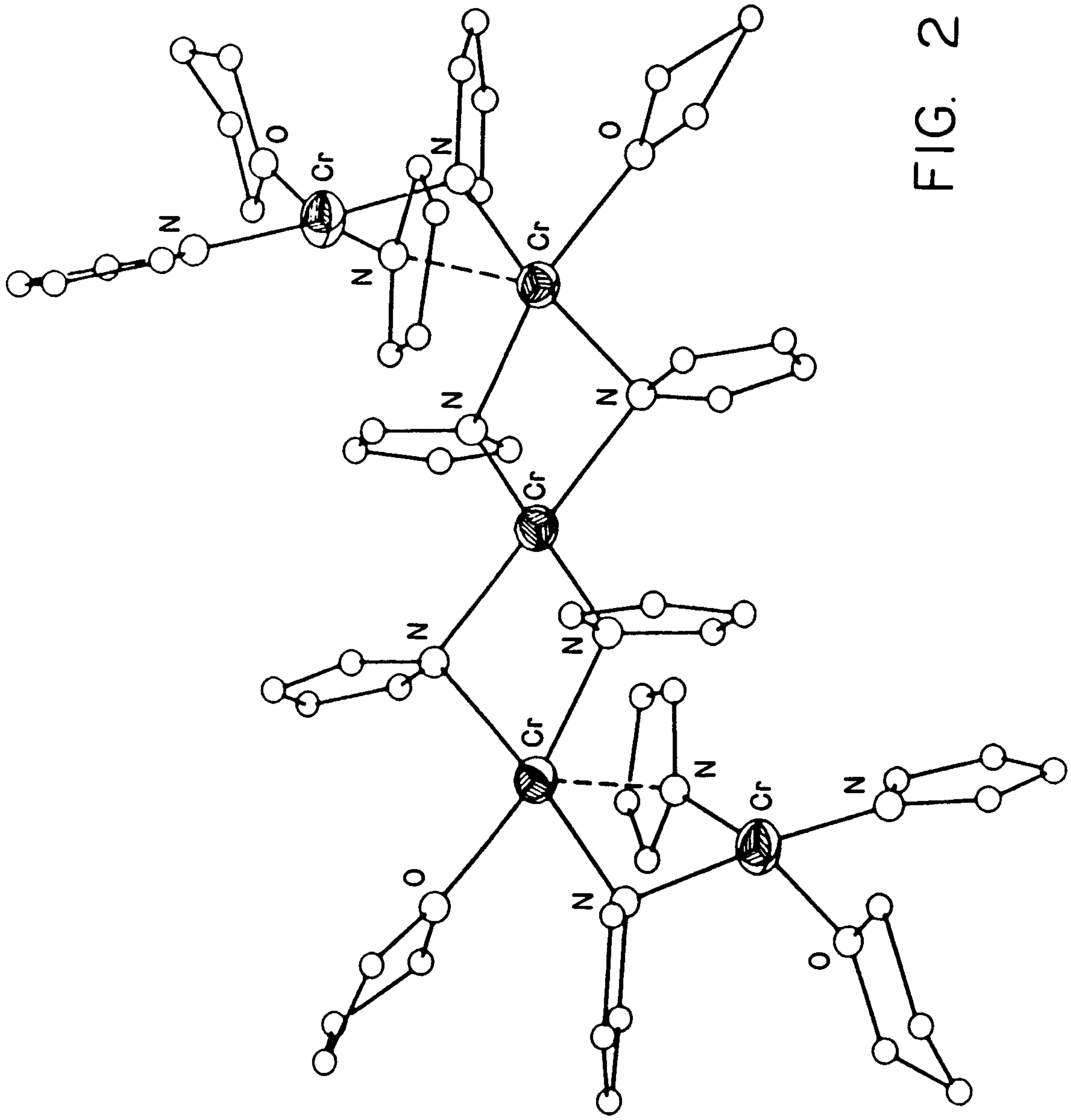

FIG. 2

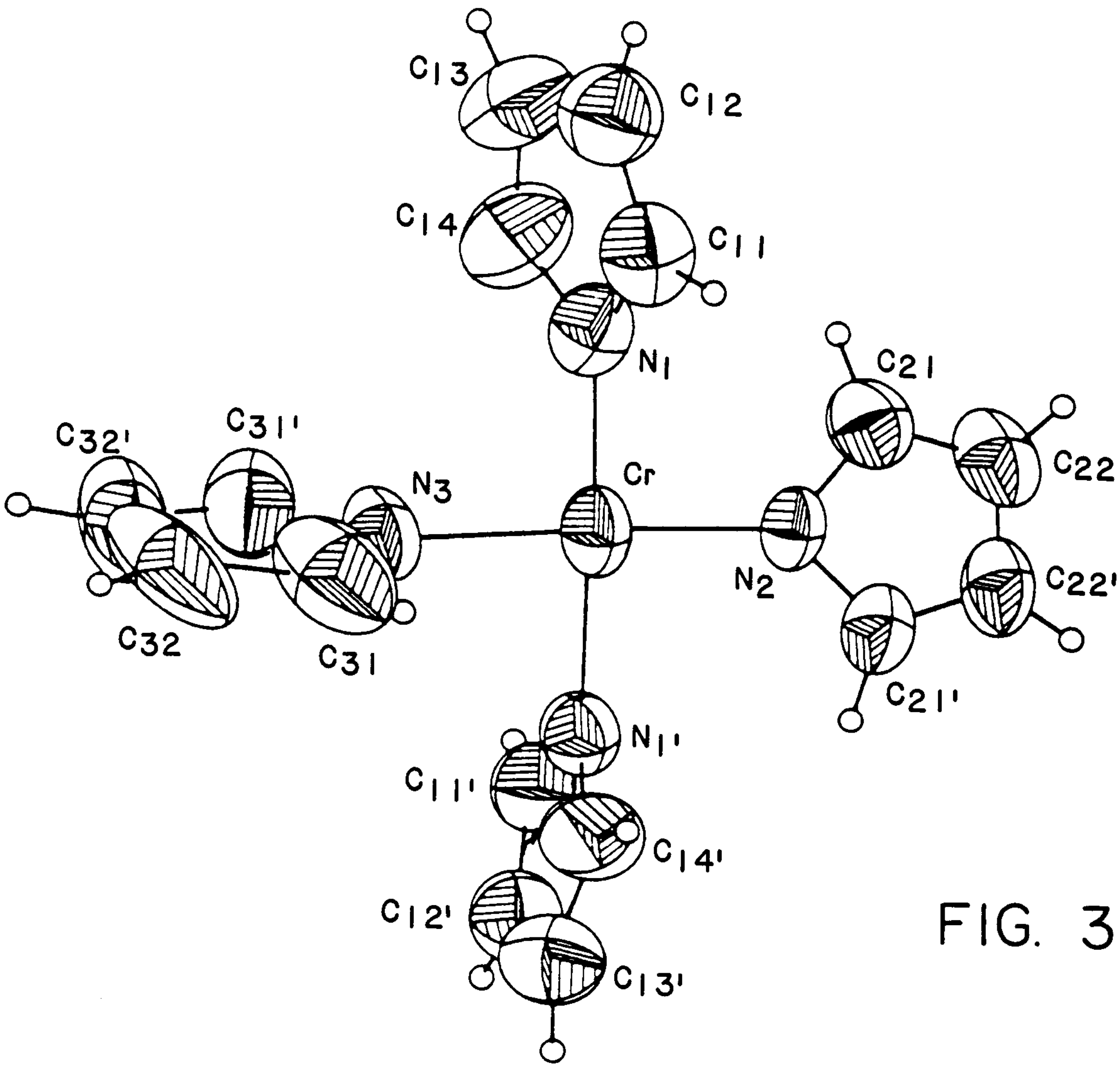

FIG. 3

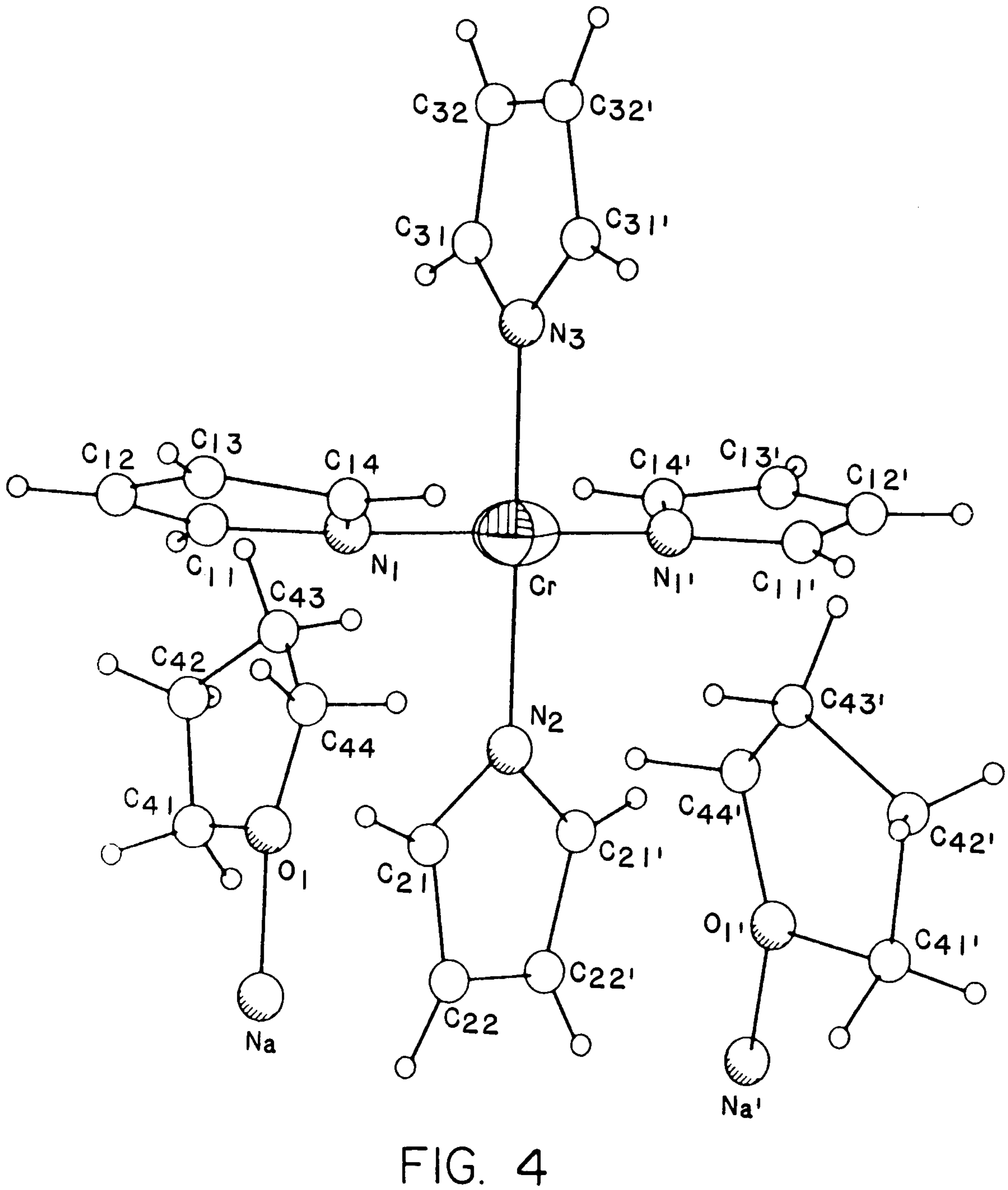

FIG. 4

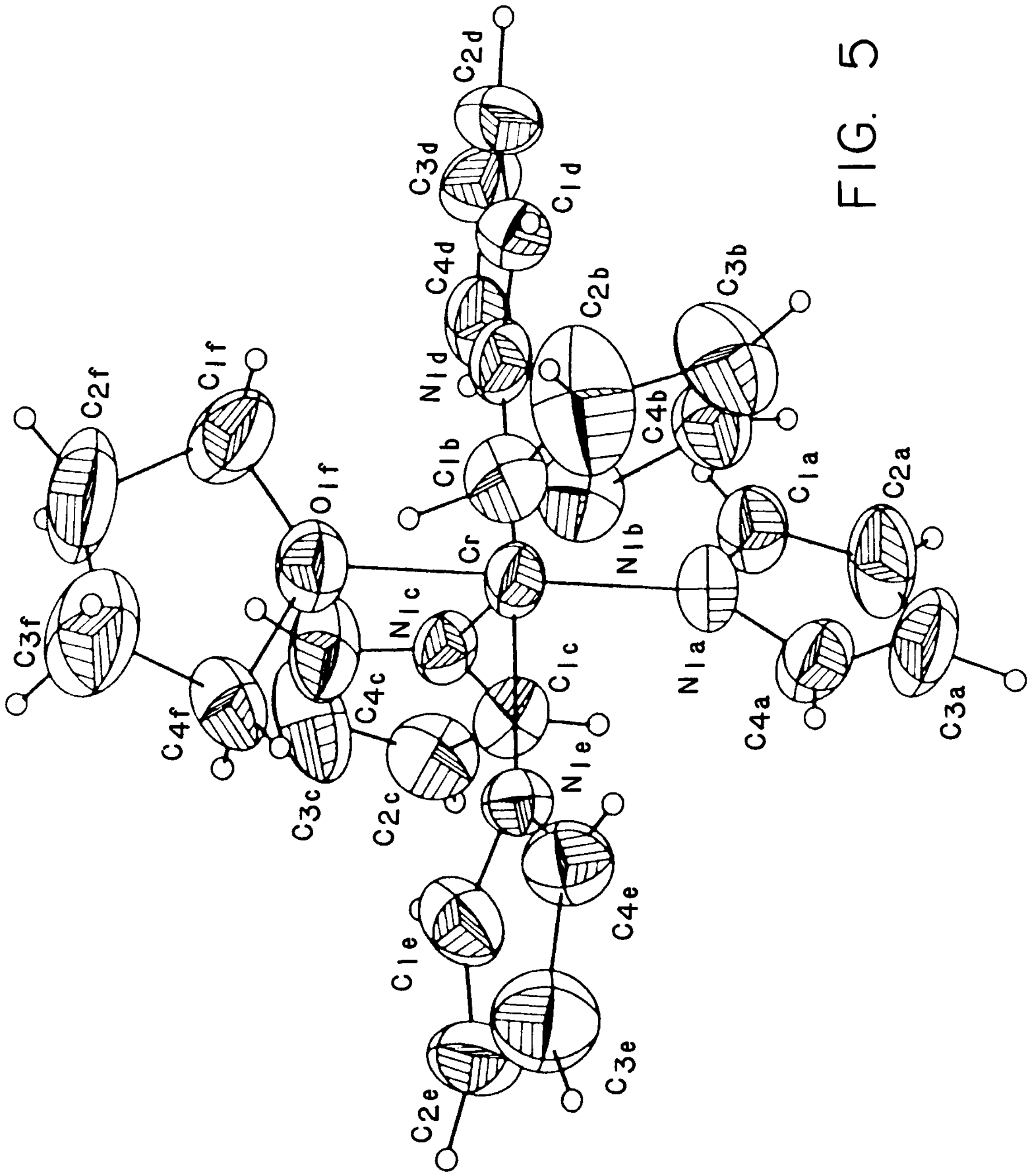

FIG. 5

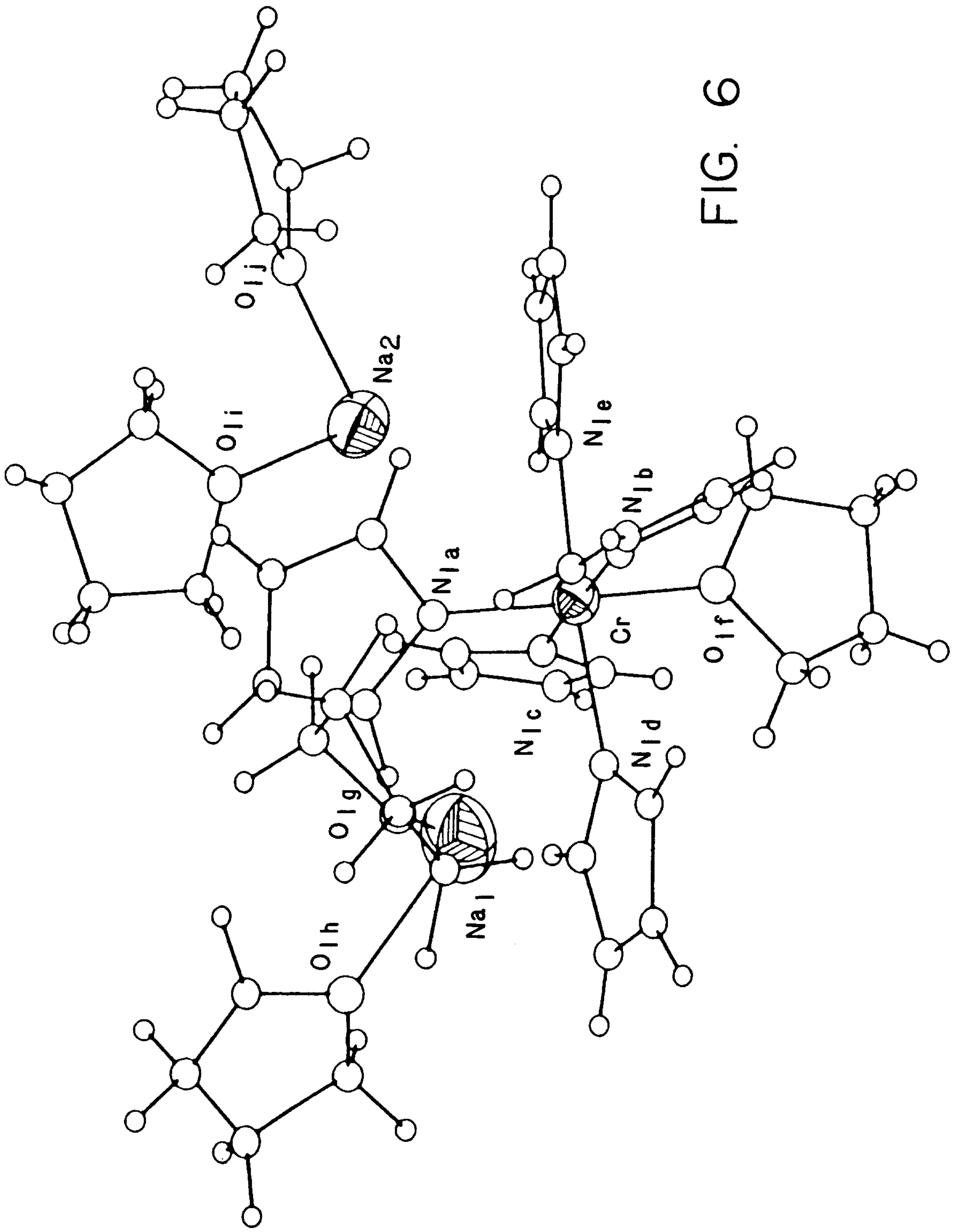

FIG. 6